# EUROPÄISCHE PATENTSCHRIFT

(11) **EP 1 085 807 B1**
(45) Veröffentlichungstag und Bekanntmachung des Hinweises auf die Patenterteilung: **07.05.2003**
(21) Anmeldenummer: 99927805.4
(22) Anmeldetag: 29.05.1999
(51) Int. Cl.: A01N 43/54

(54) **VERWENDUNG VON 2-(N-PHENYLAMINO)PYRIMIDINEN ALS FUNGIZIDE SOWIE NEUE 2-(N-PHENYLAMINO)PYRIMIDINE**
USE OF 2-(N-PHENYLAMINO)PYRIMIDINES AS FUNGICIDES, AND NOVEL 2-(N-PHENYLAMINO)PYRIMIDINES
UTILISATION DE 2-(N-PHENYLAMINO)PYRIMIDINES COMME FONGICIDES ET NOUVELLES 2-(N-PHENYLAMINO)PYRIMIDINES

(30) Priorität: 10.06.1998 DE 19825803
(43) Veröffentlichungstag der Anmeldung: 28.03.2001
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); BAYER, Herbert, D-68159 Mannheim (DE); CULLMANN, Oliver, D-68199 Mannheim (DE); GEWEHR, Markus, D-56288 Kastellaun (DE); GRAMMENOS, Wassilios, D-67063 Ludwigshafen (DE); GYPSER, Andreas, D-68159 Mannheim (DE); PTOCK, Arne, D-67067 Ludwigshafen (DE); GÖTZ, Norbert, D-67547 Worms (DE); GROTE, Thomas, D-67105 Schifferstadt (DE); RACK, Michael, D-69123 Heidelberg (DE); AMMERMANN, Eberhard, D-64646 Heppenheim (DE); LORENZ, Gisela, D-67434 Neustadt (DE); STRATHMANN, Siegfried, D-67117 Limburgerhof (DE); SPEAKMAN, John-Bryan, D-67273 Bobenheim (DE)
(86) Internationale Anmeldenummer: EP9903751
(87) Internationale Veröffentlichungsnummer: WO99063821

(56) Entgegenhaltungen:
- EP-A- 0 172 786
- EP-A- 0 270 111
- EP-A- 0 337 944
- EP-A- 0 457 726
- PATENT ABSTRACTS OF JAPAN vol. 016, no. 081 (C-0915), 27. Februar 1992 (1992-02-27) & JP 03 271278 A (NISSAN CHEM IND LTD), 3. Dezember 1991 (1991-12-03) in der Anmeldung erwähnt

## Beschreibung

Die vorliegende Erfindung betrifft die Verwendung von 2-(N-Phenylamino)pyrimidinen der Formel I, 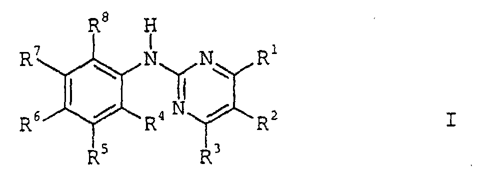 in der die Substituenten die folgende Bedeutung haben:
- R¹, R³: unabhängig voneinander Cyano, C₁-C₈-Alkyl, C₂-C₆-Alkenyl, C₂-C₆-Alkinyl, wobei die Reste Alkyl, Alkenyl und Alkinyl durch Cyano, Halogen, C₁-C₄-Alkoxy oder C₁-C₄-Alkoxycarbonyl substituiert sein können, C₃-C₈-Cycloalkyl oder eine Gruppe C(=NOR^{x})R^{y}
- R^{x}: Wasserstoff, C₁-C₈-Alkyl, C₂-C₆-Alkenyl, C₂-C₆-Alkinyl, wobei die Reste Alkyl, Alkenyl und Alkinyl durch Cyano, Halogen, C₁-C₄-Alkoxy oder Phenyl substituiert sein können;
- R^{y}: Wasserstoff, C₁-C₈-Alkyl, C₂-C₆-Alkenyl, C₂-C₆-Alkinyl, wobei die Reste Alkyl, Alkenyl und Alkinyl durch Cyano, Halogen oder C₁-C₄-Alkoxy substituiert sein können.
- R²: Halogen, C₁-C₈-Alkyl, C₂-C₆-Alkenyl, C₂-C₆-Alkinyl, wobei die Reste Alkyl, Alkenyl und Alkinyl durch Cyano, Halogen, C₁-C₄-Alkoxy oder C₁-C₄-Alkoxycarbonyl substituiert sein können, oder
- R⁴ bis R⁸: unabhängig voneinander Wasserstoff, Cyano, Halogen, R^{a}, R^{a}O, R^{a}S(O)ₘ, R^{a}O-(C=O), R^{a}(C=O), R^{a}R^{b}N-(C=O), R^{a}HN-(C=O),H₂N-(C=O), R^{a}-(C=O)-NH, R^{a}-(C=O)-NR^{b} oder R^{a}O-N=C(R^{b}) ;
- R^{a}, R^{b}: C₁-C₄-Alkyl, C₃-C₆-Alkenyl oder C₃-C₆-Alkinyl, welche jeweils durch Cyano, Halogen, C₁-C₄-Alkoxy, C₁-C₄-Alkoxycarbonyl oder Phenyl substituiert sein können oder Phenyl, welches ein bis drei Substituenten ausgewählt aus der Gruppe: Halogen und C₁-C₄-Alkyl tragen kann;
- m: 0, 1 oder 2;
als Fungizide.

Aus der EP-A 224 339, EP-A 270 111, EP-A 310 550, EP-A 457 726, DD 151404 und JP 03/271278 sind fungizide 2-(N-Phenylamino)pyrimidine bekannt, die in 5-Stellung am Pyrimidinring ein Wasserstoffatom tragen.

In 5-Stellung substituierte 2-(N-Phenylamino)pyrimidine sind bisher als Zwischenprodukte für herbizide Wirkstoffe (EP-A 337 944) und als Pharmazeutika (WO-A 97/19065) beschrieben.

Schließlich sind aus der EP-A 172 786 fungizide 2-(N-Phenylamino)pyrimidine bekannt, die in 5-Stellung substituiert sein können und an der Aminofunktion einen speziellen 2-Nitrophenylrest tragen.

Die in der EP-A 172 786 genannten Verbindungen können jedoch die in der Praxis an Wirkstoffe gestellten Forderungen nicht immer voll befriedigen.

Aufgabe der vorliegenden Erfindung war es, fungizide Wirkstoffe mit verbesserten Eigenschaften zu finden.

Demgemäß wurde gefunden, daß sich die eingangs erwähnten Verbindungen, die z. Teil bereits in den Schriften EP-A 337 944 und WO-A 97/19065 erwähnt wurden, hervorragend zur Bekämpfung von Schadpilzen eignen. Darüberhinaus wurden neue 2-(N-Phenylamino)pyrimidine mit verbesserter fungizider Wirkung gefunden.

Gemeinsam ist allen erfindungsgemäßen Aminopyrimidinen, den neuen wie den aus EP-A 337 944 und WO-A 97/19065 bekannten, daß sie in 4-, 5- und 6-Stellung jeweils einen hydrophoben Substituenten tragen.

Die neuen 2-(N-Phenylamino)pyrimidinen können analog zu literaturbekannten Verfahren hergestellt werden. Insbesondere bieten sich die in Schema 1 und 2 aufgeführten Verfahrensrouten an.

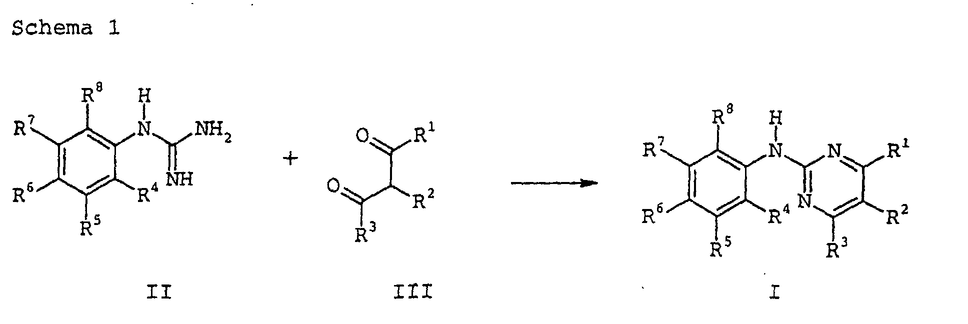

Die Kondensationreaktion der Guanidine II mit 1,3-Dicarbonylverbindungen der Formel III zu den 2-(N-Phenylamino)pyrimidinen I kann wie in der EP-A 337 944 und der dort zitierten Literatur beschrieben durchgeführt werden.

Hinsichtlich der Synthese der Guanidine II sei auf die Literaturstellen: Houben Weyl, Methoden d. Org. Chemie, Stuttgart, Bd. VIII S. 98, 180 bis 189 verwiesen. Die 1,3-Diketone III lassen sich beispielsweise entweder durch a) Claisen Kondensation oder durch b) Alkylierung bzw. Halogenierung eines in 2-Stellung unsubstituierten 1,3-Diketons herstellen (Organikum, 1993 Barth Verlagsgesellschaft Leipzig, a) S.487 b) S. 536).

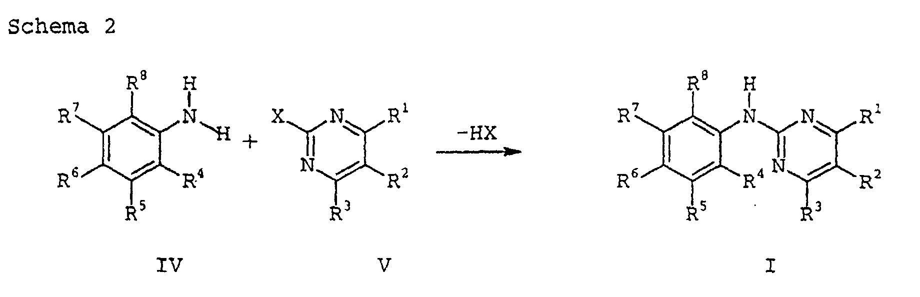

Die Herstellung der 2-(N-Phenylamino)pyrimidinen I ausgehend von Anilinderivaten der Formel IV und Pyrimidinderivaten der Formel V, wobei X für eine nukleophil austauschbare Gruppe wie Halogen oder C₁-C₄-Alkylsulfonyl steht, ist in der EP-A 337 944 beschrieben.

Die erfindungsgemäßen Verbindungen I, in denen R³ C(=NOR^{x})R^{y} bedeutet, werden bevorzugt aus den Aldehyden VI hergestellt (s. Schema 3), wobei die Aldehyde VI ihrerseits analog EP-A 457 726 synthetisiert werden können.

Verbindungen Ia, in denen R³ CH(=NOR^{x}) bedeutet, werden durch Umsetzung der Aldehyde VI mit den Alkoxyaminen VII unter an sich bekannten Bedingungen erhalten (Schema 3).

Außerdem können die Aldehyde VI unter an sich bekannten Bedingungen mit metallorganischen Reagenzien, wie z. B. Grignard-Verbindungen VIII (R^{y}-Mg-X; X = Cl, Br, I) zu den sekundären Alkoholen IX umgesetzt werden.

Die Oxidation dieser Alkohole IX, vorzugsweise nach Swern mit Oxalylchlorid/DMSO, liefert die Ketone X, die ihrerseits mit Alkoxyaminen VII zu den erfindungsgemäßen Verbindungen Ib umgesetzt werden können.

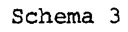

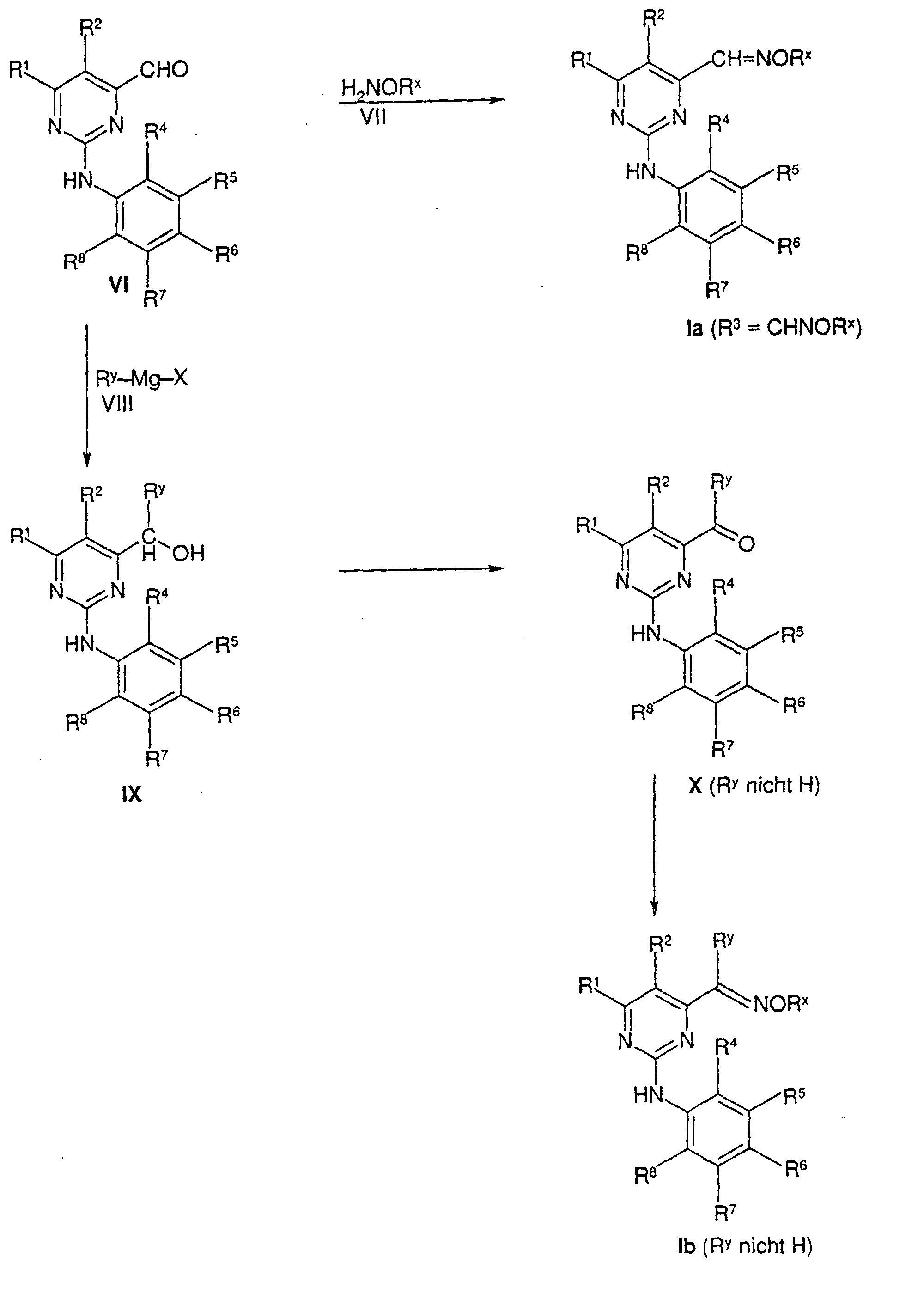

Die Wirkstoffe Ic, bei denen R³ Cyano bedeutet (Schema 4), können bevorzugt durch Umsetzung der Aldedhyde VI mit Hydroxylamin und Dehydratisierung der so gebildeten Oxime XI hergestellt werden (analog EP-A 457 726).

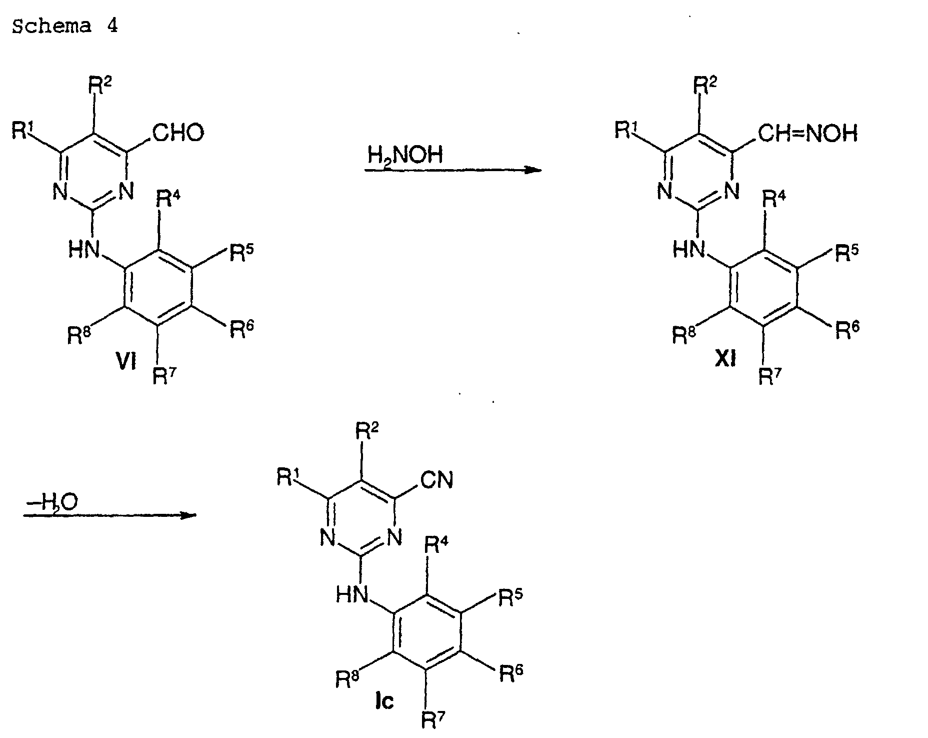

Bei der eingangs angegebenen Definition der Verbindungen I wurden für die Reste R¹ bis R⁸, R^{x} und R^{y} sowie R^{a} und R^{b} Sammelbegriffe verwendet, die für individuelle Aufzählungen der einzelnen Gruppenmitglieder stehen. Die Reste Alkyl, Alkoxy, Alkoxycarbonyl, Alkenyl und Alkinyl können geradkettig oder verzweigt sein.

Beispielsweise bedeuten:
- Halogen: Fluor, Chlor, Brom oder Jod;
- C₁-C₄-Alkyl: Methyl, Ethyl, Propyl, 1-Methylethyl, Butyl, 1-Methylpropyl, 2-Methylpropyl oder 1,1-Dimethylethyl;
- C₁-C₈-Alkyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl, 1-Ethyl-3-methylpropyl, Heptyl, Octyl oder 2-Ethylhexyl;
- C₁-C₂-Halogenalkyl: einen C₁-C₂-Alkylrest wie vorstehend genannt, der partiell oder vollständig durch Fluor, Chlor, Brom und/oder Jod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Jodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl oder Pentafluorethyl;
- C₁-C₄-Alkoxy sowie die Alkoxyteile von C₁-C₄-Alkoxycarbonyl : Methoxy, Ethoxy, Propoxy, 1-Methylethoxy, Butoxy, 1-Methylpropoxy, 2-methylpropoxy oder 1,1-Dimethylethoxy;
- C₁-C₂-Halogenalkoxy: einen C₁-C₂-Alkoxyrest wie voranstehend genannt, der partiell oder vollständig durch Fluor, Chlor, Brom und/oder Jod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Jodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy;
- C₂-C₆-Alkenyl: Ethylen,Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methylprop-2-en-1-yl und 2-Methyl-prop-2-en-1-yl, Penten-1-yl, Penten-2-yl, Penten-3-yl, Penten-4-yl, 1-Methylbut-1-en-1-yl, 2-Methyl-but-l-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-nent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimetyl-but-2-en-1-yl, 1,2-Dimethyl-but-3-en-1-yl, 1,3-Dimetyl-but-l-en-1-yl, 1,3-Dimethyl-but-2-en-1-yl, 1,3-Dimetyl-but-3-en-1-yl, 2,2-Dimethyl-but-3-en-1-yl, 2,3-Dimetyl-but-1-en-1-yl, 2,3-Dimethyl-but-2-en-1-yl, 2,3-Dimetyl-but-3-en-1-yl, 3,3-Dimethyl-but-1-en-1-yl, 3,3-Dimetyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl oder 1-Ethyl-2-methyl-prop-2-en-1-yl;
- C₂-C₆-Alkinyl : Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl,4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl oder 1-Ethyl-1-methyl-2-propinyl;
- C₃-C₈-Cycloalkyl: Cyclopropyl, Cyclobutyl, Cyclopentyl, Cyclohexyl, Cycloheptyl oder Cyclooctyl;

Hinsichtlich ihrer Verwendung als Fungizide sind 2-(N-Phenylamino)pyrimidine I mit folgenden Substituenten bevorzugt, wobei die Bevorzugung jeweils für sich allein oder in Kombination zu sehen ist:
- R¹, R³: Cyano, C₁-C₈-Alkyl, Propinyl, C₁-C₂-Haloalkyl, Cyclopropyl oder eine Gruppe C(=NOR^{x})R^{y} ;
- R²: Halogen oder C₁-C₈-Alkyl ;
- R⁴ bis R⁸: unabhängig voneinander wasserstoff, Cyano, Halogen, C₁-C₂-Haloalkyl, C₁-C₂-Haloalkoxy, R^{a} oder R^{a}O-N=C(R^{b}) ;
- R^{a}, R^{b}: C₁-C₄-Alkyl.

Insbesondere bevorzugt sind 2-(N-Phenylamino)pyrimidine I mit folgenden Substituenten, wobei die Bevorzugung jeweils für sich allein oder in Kombination zu sehen ist:
- R¹, R³: Cyano, Methyl, Ethyl, Cyclopropy; CH (=NOC₂H₅), C(=NOCH₃)CH₃ oder C(=NOC₂H₅)CH₃;
- R²: Fluor, Chlor, Methyl oder Ethyl;
- R⁴ bis R⁸: unabhängig voneinander Wasserstoff, Cyano, Halogen oder C¹-C⁴-Alkyl.

Am meisten bevorzugt sind 2-(N-Phenylamino)pyrimidine I mit folgenden Substituenten, wobei die Bevorzugung jeweils für sich allein oder in Kombination zu sehen ist.
- R¹: Methyl oder Cyclopropy;
- R²: Methyl oder Fluor;
- R³: C(=NOCH₃), C(=NOC₂H₅), C(=NOCH₃)CH₃, C(=NOC₂H₅)CH₃, Cyano oder Methyl und
- R⁴ bis R⁸: Wasserstoff.

Folgende Wirkstoffgruppen sind aufgrund ihrer ausgeprägten pflanzenfungiziden Aktivität bevorzugt.

Gruppe Ia: 2-(N-Phenylamino)pyrimidine der Formel I, in der
- R¹, R³: Cyano, C₁-C₈-Alkyl, C₂-C₆-Alkenyl, C₂-C₆-Alkinyl oder C₃-C₈-Cycloalkyl;
- R²: Halogen, C₁-C₈-Alkyl, C₂-C₆-Alkenyl, C₂-C₆-Alkinyl oder C₁-C₂-Haloalkyl ;
- R⁴,R⁶,R⁸: unabhängig voneinander Wasserstoff, Cyano, Halogen, R^{a}, R^{a}O, R^{a}S(O)ₘ, R^{a}O-(C=O), R^{a}(C=O), R^{a}R^{b}N-(C=O), R^{a}HN-(C=O),H₂N-(C=O), R^{a}-(C=O)-NH, R^{a}-(C=O)-NR^{b} oder R^{a}O-N=C (R^{b}) ;
- R⁵, R⁷: unabhängig voneinander Wasserstoff, Cyano, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R¹-(C=O)-NH, R^{a}-(C=O)-NR^{b} oder R^{a}O-N=C(R^{b}) ;
- R^{a}, R^{b}: C₁-C₄-Alkyl, C₃-C₆-Alkenyl oder C₃-C₆-Alkinyl, welche jeweils durch Cyano, Halogen, C₁-C₄-Alkoxy, C₁-C₄-Alkoxycarbonyl oder Phenyl substituiert sein können oder Phenyl, welches ein bis drei Substituenten ausgewählt aus der Gruppe: Halogen und C₁-C₄-Alkyl tragen kann, und
- m: 0,1 oder 2 bedeuten.

Bevorzugt sind 2-(N-Phenylamino)pyrimidine der Gruppe Ia, in der
- R¹: Cyano, Methyl, Ethyl, n-Propyl, Propinyl oder Cyclopropyl ;
- R²: Methyl, Ethyl, Fluor oder Chlor;
- R³: Methyl ;
- R⁴,R⁶,R⁸: unabhängig voneinander Wasserstoff, Cyano, Halogen, C₁-C₂-Haloalkoxy, R^{a} oder R^{a}O-N=C(R^{b}) und vorzugsweise Wasserstoff, Halogen oder C₁-C₄-Alkyl ;
- R⁵,R⁷: unabhängig voneinander Wasserstoff, Cyano, C₁-C₂-Haloalkoxy, R^{a} oder R^{a}O-N=C(R^{b}) und vorzugsweise Wasserstoff, Halogen oder C₁-C₄-Alkyl und
- R^{a}, R^{b}: C₁-C₄-Alkyl bedeuten.

Gruppe Ib: 2-(N-Phenylamino)pyrimidine der Formel I, in der
- R¹: Cyano oder C₂-C₆-Alkinyl und
die Reste R² bis R⁸ die in Anspruch 1 angegebene Bedeutung haben.

Bevorzugt sind 2-(N-Phenylamino)pyrimidine der Gruppe Ib, in der
- R¹: Cyano oder Propinyl;
- R²: Halogen oder C₁-C₈-Alkyl und vorzugweise Fluor, Chlor, Methyl oder Ethyl;
- R³: Cyano, C₁-C₈-Alkyl, Propinyl, C₁-C₂-Haloalkyl oder Cyclopropyl und vorzugweise Methyl, Ethyl, n-Propyl, Propinyl oder Cyclopropyl;
- R⁴ bis R⁸: unabhängig voneinander Wasserstoff, Cyano, Halogen, C₁-C₂-Haloalkyl, C₁-C₂-Haloalkoxy, R^{a} oder R^{a}O-N=C(R^{b}) und vorzugsweise Wasserstoff, Halogen oder C₁-C₄-Alkyl und
- R^{a}, R^{b}: C₁-C₄-Alkyl bedeuten.

Gruppe Ic: 2-(N-Phenylamino)pyrimidine der Formel I, in der
- R⁴ bis R⁸: Wasserstoff und
die Reste R¹ bis R³ die in Anspruch 1 angegebene Bedeutung haben.

Bevorzugt sind 2-(N-Phenylamino)pyrimidine der Gruppe Ic, in der
- R¹, R³: Cyano, C₁-C₈-Alkyl, Propinyl, C₁-C₂-Haloalkyl oder Cyclopropyl und vorzugweise Methyl, Ethyl, n-Propyl, Propinyl oder Cyclopropyl;
- R²: Halogen oder C₁-C₈-Alkyl und vorzugweise Fluor, Chlor, Methyl oder Ethyl und
- R⁴ bis R⁸: Wasserstoff bedeuten.

Gruppe Id: 2-(N-Phenylamino)pyrimidine der Formel I, in der
- R²: Halogen;
- R⁴, R⁸: unabhängig voneinander Wasserstoff, Cyano, Halogen, C₁-C₂-Haloalkoxy, R^{a}, R^{a}O, R^{a}S(O)ₘ, R^{a}O-(C=O), R^{a}(C=O), R^{a}R^{b}N-(C=O), R^{a}HN-(C=O),H₂N-(C=O), R^{a}-(C=O) -NH, R^{a}-(C=O)-NR^{b} oder R^{a}O-N=C(R^{b}) ;
- R⁵ bis R⁷: unabhängig voneinander Wasserstoff, Cyano, Halogen, C₁-C₂-Haloalkyl, C₁-C₂-Haloalkoxy, R^{a}, R^{a}O, R^{a}S(O)ₘ, R^{a}O-(C=O), R³(C=O), R^{a}R^{b}N-(C=O), R^{a}HN- (C=O),H₂N-(C=O), R^{a}-(C=O)-NH, R^{a}-(C=O)-NR^{b} oder R^{a}ON=C(R^{b}) ;
- R^{a}, R^{b}: C₁-C₄-Alkyl, C₃-C₆-Alkenyl oder C₃-C₆-Alkinyl, welche jeweils durch Cyano, C₁-C₄-Alkoxy, C₁-C₄-Alkoxycarbonyl oder Phenyl substituiert sein können oder
Phenyl, welches ein bis drei Substituenten ausgewählt aus der Gruppe: Halogen und C₁-C₄-Alkyl tragen kann, und
die Reste R¹ und R³ die in Anspruch 1 angegebene Bedeutung haben.

Bevorzugt sind 2-(N-Phenylamino)pyrimidine der Gruppe Id, in der
- R¹, R³: Cyano, C₁-C₈-Alkyl, Propinyl, C₁-C₂-Haloalkyl oder Cyclopropyl und vorzugweise Methyl, Ethyl, n-Propyl, Propinyl oder Cyclopropyl;
- R²: Fluor oder Chlor;
- R⁴, R⁸: unabhängig voneinander Wasserstoff, Cyano, Halogen, C₁-C₂-Haloalkoxy, R^{a} oder R^{a}O-N=C(R^{b}) und vorzugsweise Wasserstoff, Halogen oder C₁-C₄-Alkyl ;
- R⁵ bis R⁷: unabhängig voneinander Wasserstoff, Cyano, Halogen, C₁-C₂-Haloalkyl, C₁-C₂-Haloalkoxy, R^{a} oder R^{a}O-N=C(R^{b}) und vorzugsweise Wasserstoff, Halogen oder C₁-C₄-Alkyl und
- R^{a}, R^{b}: C₁-C₄-Alkyl bedeuten.

Gruppe Ie: 2-(N-Phenylamino)pyrimidine der Formel I, in der
- R¹, R³: C₁-C₈-Alkyl, C₂-C₆-Alkenyl oder C₂-C₆-Alkinyl, wobei die Reste Alkenyl und Alkinyl durch Cyano, Halogen, C₁-C₄-Alkoxy oder C₁-C₄-Alkoxycarbonyl substituiert sein können;
- R²: C₁-C₈-Alkyl, C₂-C₆-Alkenyl oder C₂-C₆-Alkinyl ;
- R⁵, R⁷: unabhängig voneinander Wasserstoff, Cyano, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} oder R^{a}O-N=C(R^{b}) ;
die Reste R⁴, R⁶ und R⁸ die in Anspruch 1 angegebene Bedeutung haben.

Bevorzugt sind 2-(N-Phenylamino)pyrimidine der Gruppe Ie, in der
- R¹, R³: Methyl, Ethyl, n-Propyl oder Propinyl;
- R²: Methyl, Ethyl oder n-Propyl;
- R⁴,R⁶,R⁸: unabhängig voneinander Wasserstoff, Cyano, Halogen, C₁-C₂-Haloalkyl, C₁-C₂-Haloalkoxy, R^{a} oder R^{a}O-N=C(R^{b}) und vorzugsweise Wasserstoff, Halogen oder C₁-C₄-Alkyl;
- R⁵, R⁷: unabhängig voneinander Wasserstoff, Cyano, C₁-C₂-Haloalkyl, C₁-C₂-Haloalkoxy, R^{a} oder R^{a}O-N=C(R^{b}) und vorzugsweise Wasserstoff, Cyano, C₁-C₄-Alkyl oder C₁-C₂-Haloalkyl und
- R^{a}, R^{b}: C₁-C₄-Alkyl bedeuten.

Gruppe If: 2-(N-Phenylamino)pyrimidine der Formel I, in der
- R⁴, R⁸: Wasserstoff;
- R⁵, R⁷: unabhängig voneinander Wasserstoff, Cyano, C₁-C₂-Haloalkoxy, R^{a}, R^{a}O, R^{a}S(O)ₘ, R^{a}O-(C=O), R^{a}(C=O), R^{a}R^{b}N-(C=O), R^{a}HN-(C=O), H²N-(C=O), R^{a}-(C=O)-NH, R^{a}-(C=O)-NR^{b} oder R^{a}O-N=C(R^{b}) ;
- R⁶: unabhängig voneinander Wasserstoff, Cyano, Halogen, C₁-C₂-Haloalkyl, C₁-C₂-Haloalkoxy, R^{a}, R^{a}O, R^{a}S(O)ₘ, R^{a}O-(C=O), R^{a}(C=O), R^{a}R^{b}N-(C=O), R^{a}HN-(C=O),H₂N-(C=O), R^{a}-(C=O)-NH, R^{a}-(C=O)-NR^{b} oder R^{a}O-N=C(R^{b}) ;
- R^{a}, R^{b}: C₁-C₄-Alkyl, C₃-C₆-Alkenyl oder C₃-C₆-Alkinyl, welche jeweils durch Cyano, C₁-C₄-Alkoxy, C₁-C₄-Alkoxycarbonyl oder Phenyl substituiert sein können oder
Phenyl, welches ein bis drei Substituenten ausgewählt aus der Gruppe: Halogen und C₁-C₄-Alkyl tragen kann, und
die Reste R¹ bis R³ die in Anspruch 1 angegebene Bedeutung haben.

Bevorzugt sind 2-(N-Phenylamino)pyrimidine der Gruppe If, in der
- R¹, R³: Cyano, C₁-C₈-Alkyl, Propinyl, C₁-C₂-Haloalkyl oder Cyclopropyl und vorzugweise Methyl, Ethyl, n-Propyl, Propinyl oder Cyclopropyl;
- R²: Halogen oder C₁-C₈-Alkyl und vorzugweise Fluor, Chlor, Methyl oder Ethyl;
- R⁴, R⁸: Wasserstoff;
- R⁵, R⁷: unabhängig voneinander Wasserstoff, Cyano, C₁-C₂-Haloalkoxy, R^{a} oder R^{a}O-N=C(R^{b}) und vorzugsweise Wasserstoff, Cyano, R^{a} oder R^{a}O-N=C(R^{b}) ;
- R⁶: unabhängig voneinander Wasserstoff, Cyano, Halogen, C₁-C₂-Haloalkyl, C₁-C₂-Haloalkoxy, R^{a} oder R^{a}O-N=C(R^{b}) und vorzugsweise Wasserstoff, Halogen oder C₁-C₄-Alkyl und
- R^{a}, R^{b}: C₁-C₄-Alkyl bedeuten.

Gruppe Ig: 2-(N-Phenylamino)pyrimidine der Formel I, in der
- R¹: Cyclopropyl;
- R³: Cyano, C₁-C₈-Alkyl, C₂-C₆-Alkenyl, C₂-C₆-Alkinyl, wobei die Reste Alkyl, Alkenyl und Alkinyl durch Cyano, C₁-C₄Alkoxy oder C₁-C₄-Alkoxycarbonyl substituiert sein können, oder C₃-C₈-Cycloalkyl ;
die Reste R² und R⁴ bis R⁸ die in Anspruch 1 angegebene Bedeutung haben.

Bevorzugt sind 2-(N-Phenylamino)pyrimidine der Gruppe Ig, in der
- R¹: Cyclopropyl;
- R²: Halogen oder C₁-C₈-Alkyl und vorzugweise Fluor, Chlor, Methyl oder Ethyl;
- R³: Cyano, C₁-C₈-Alkyl, Propinyl oder Cyclopropyl und vorzugweise Methyl, Ethyl, n-Propyl, Propinyl oder Cyclopropyl;
- R⁴ bis R⁸: unabhängig voneinander Wasserstoff, Cyano, Halogen, C₁-C₂-Haloalkyl, C₁-C₂-Haloalkoxy, R^{a} oder R^{a}O-N=C(R^{b}) und vorzugsweise Wasserstoff, Halogen oder C₁-C₄-Alkyl und
- R^{a}, R^{b}: C₁-C₄-Alkyl bedeuten.

Beispiele für insbesondere bevorzugte 2-(N-Phenylamino)pyrimidine sind in den folgenden Tabellen zusammengestellt. 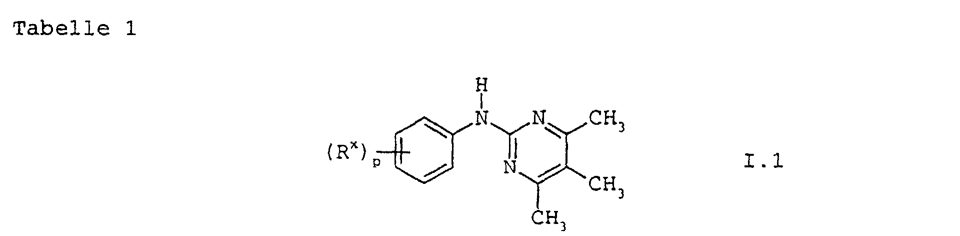 2-(N-Phenylamino)pyrimidine I.1.001 bis I.1-379 der allgemeinen Formel I.1.

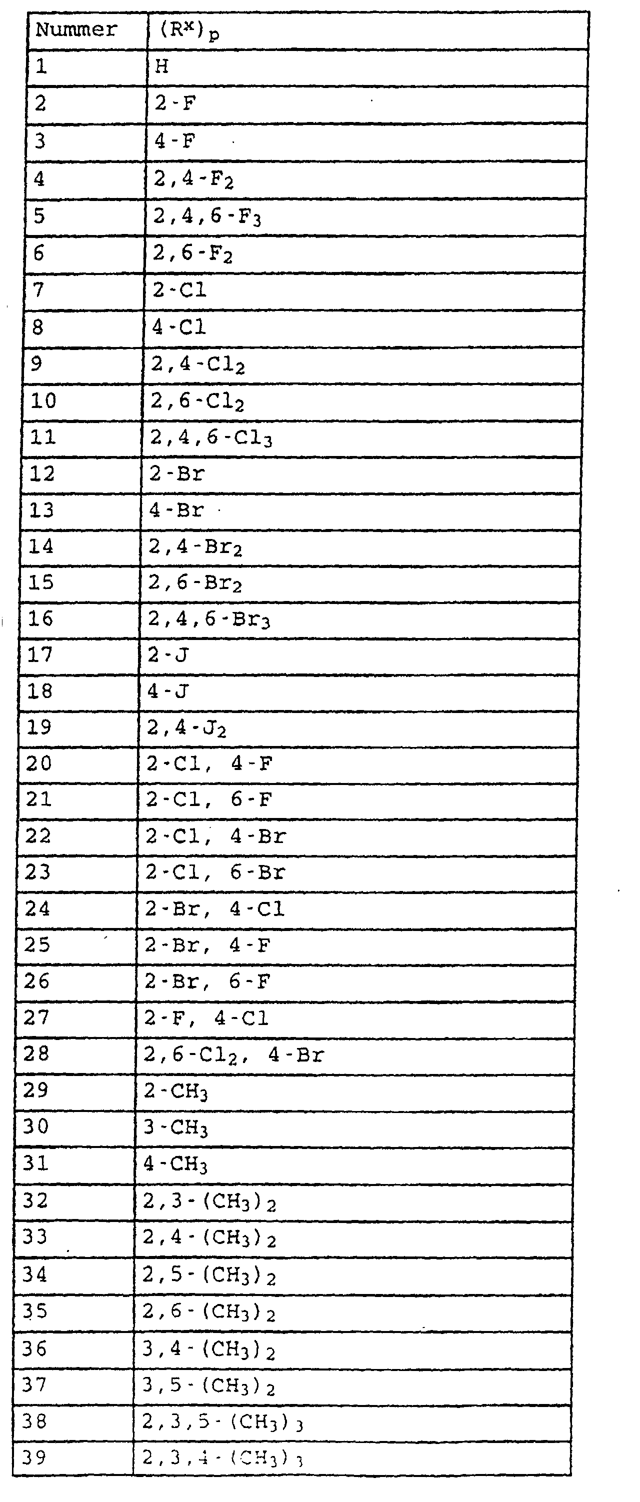

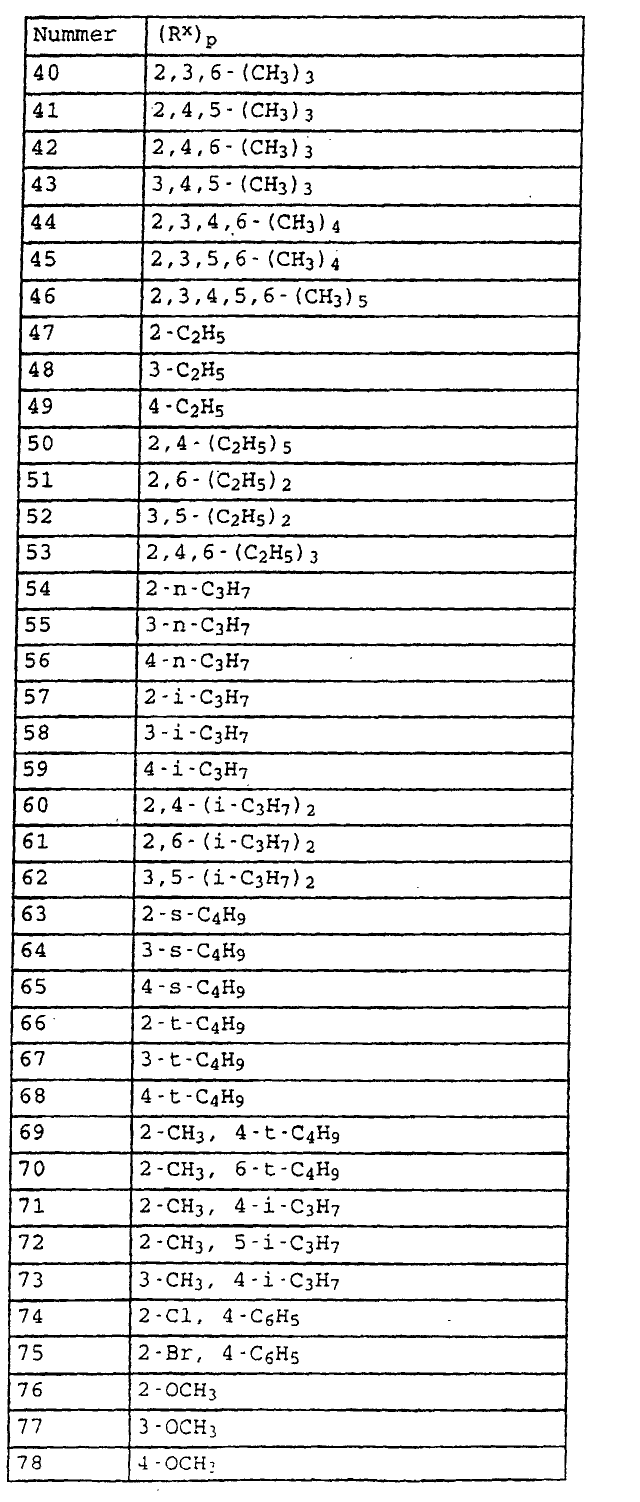

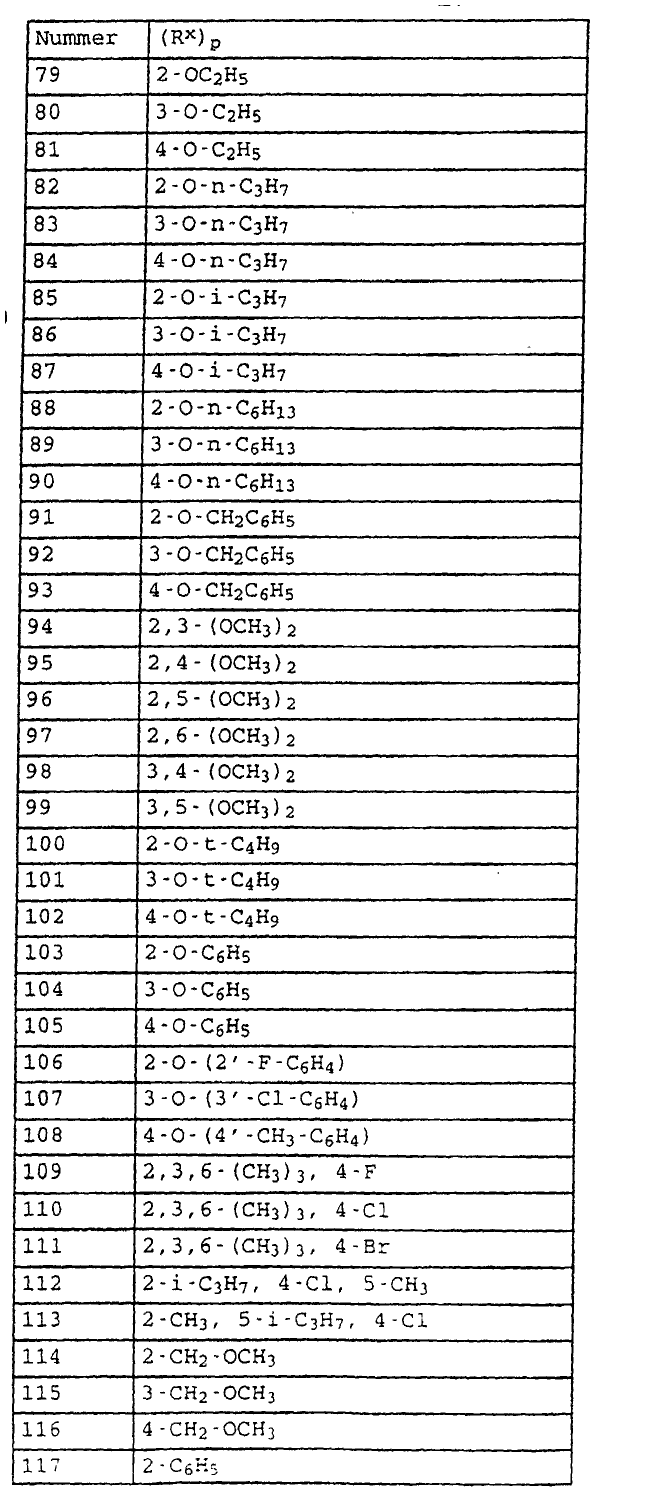

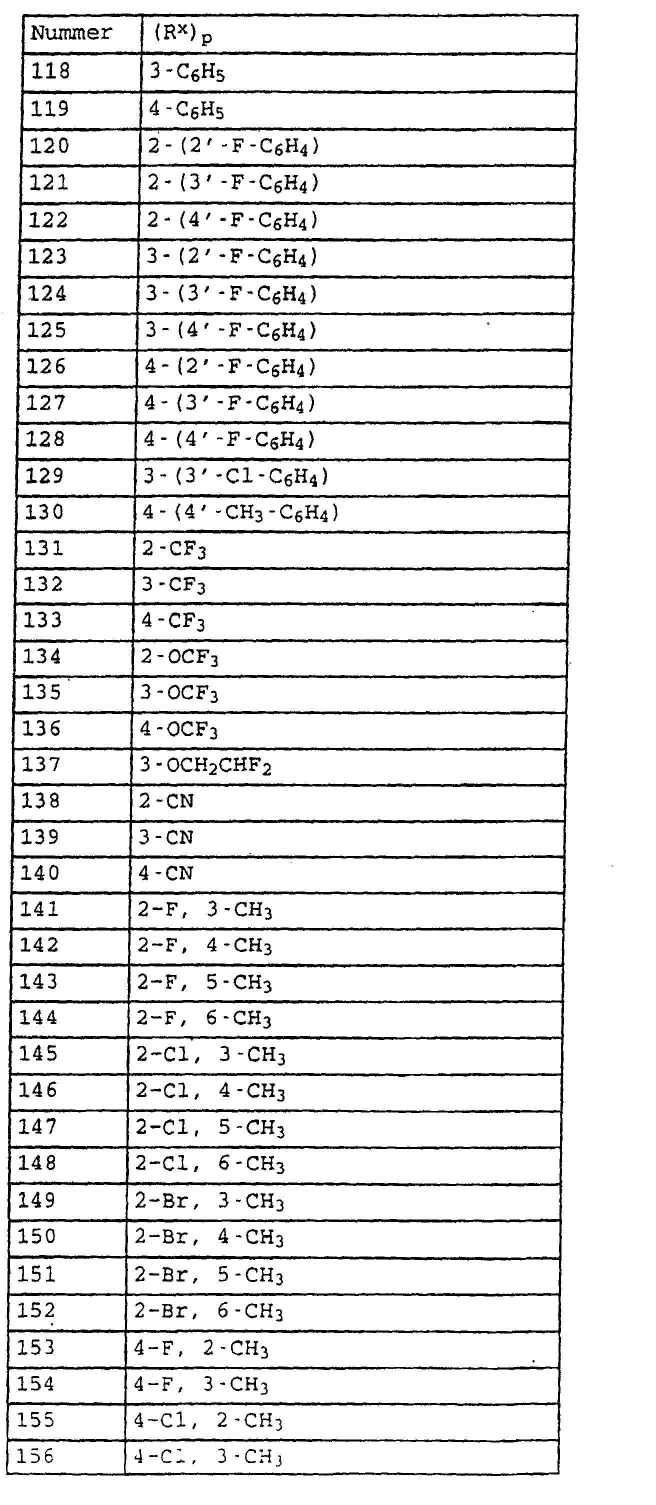

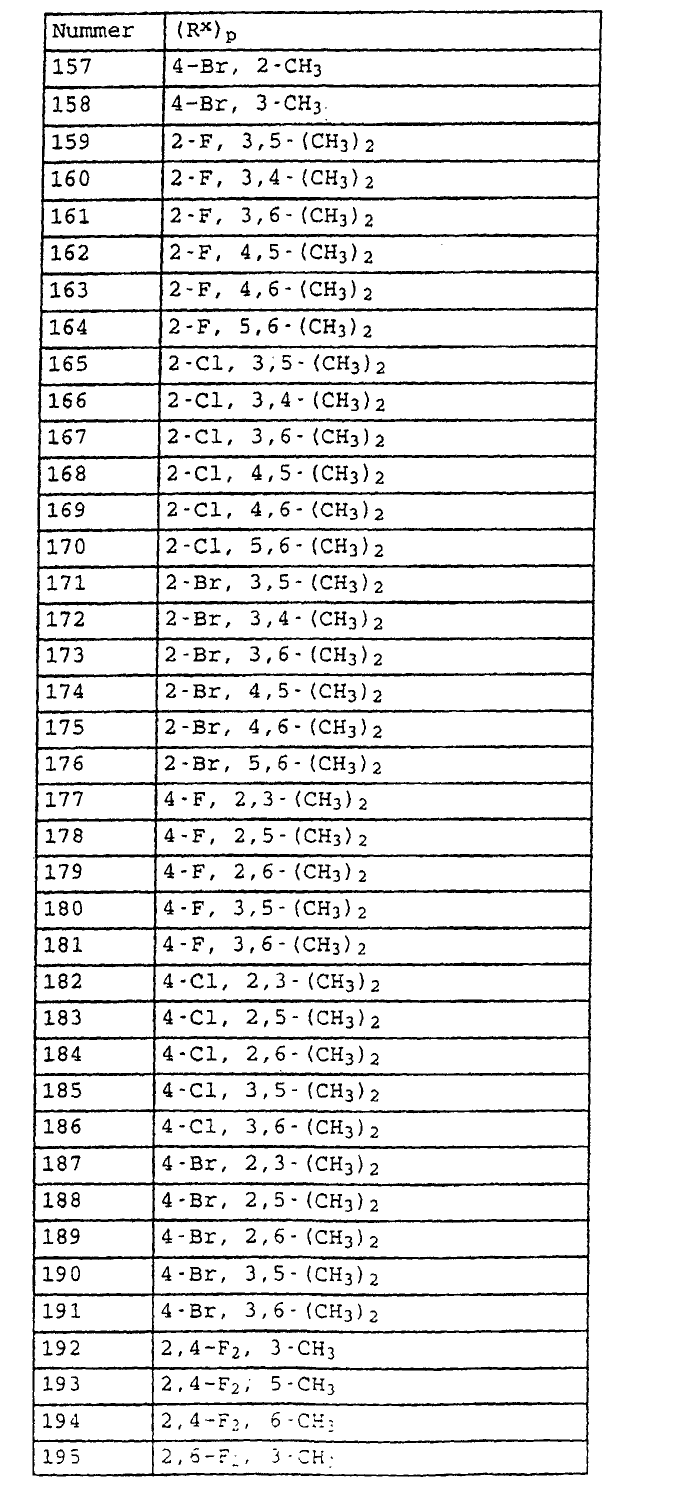

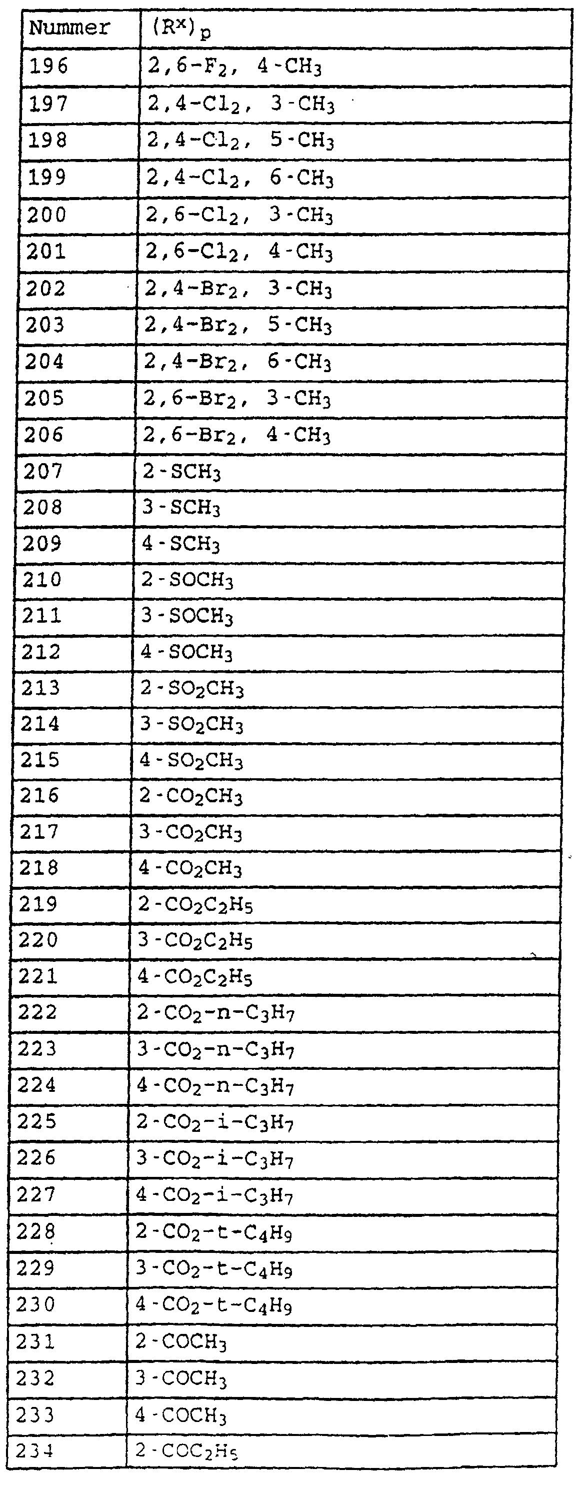

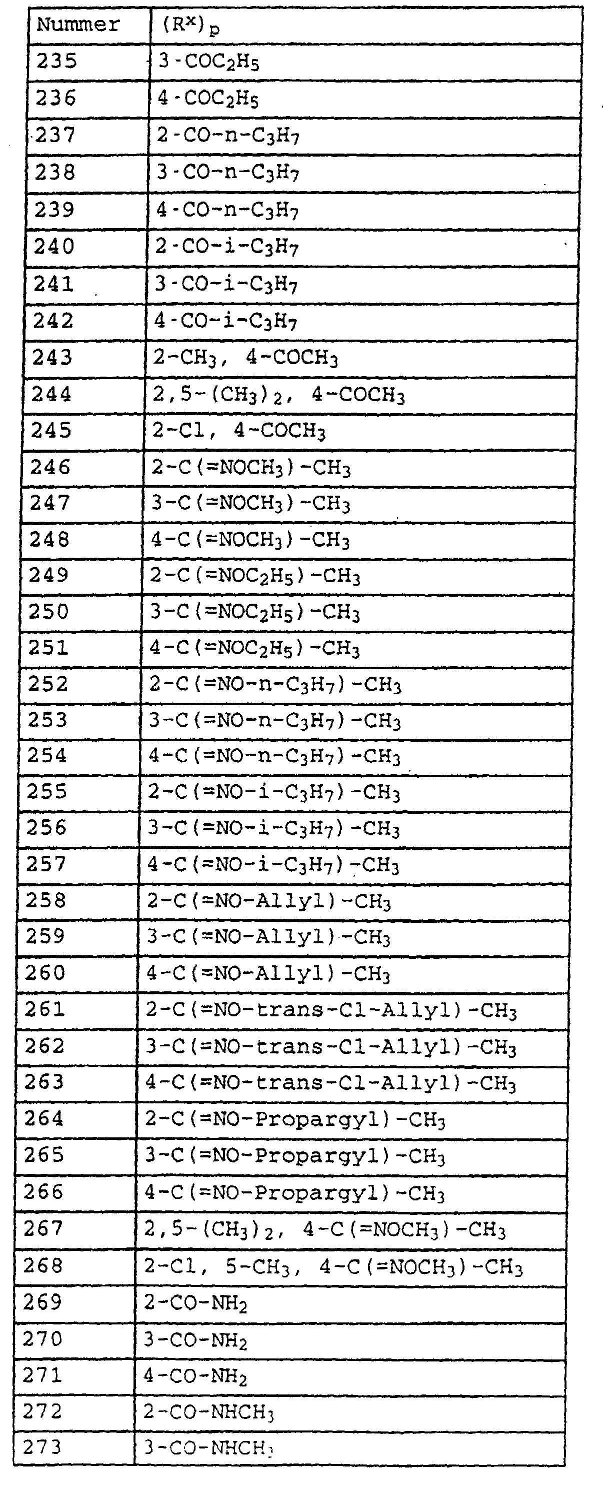

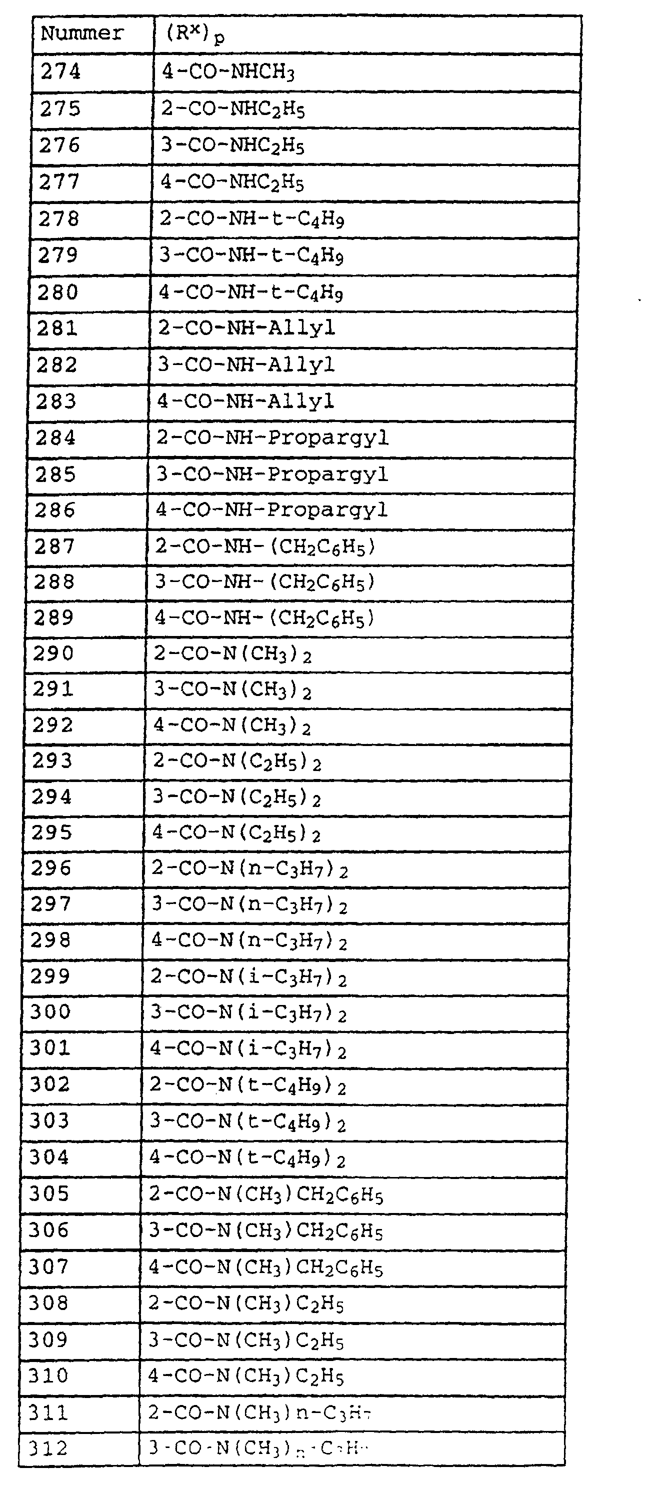

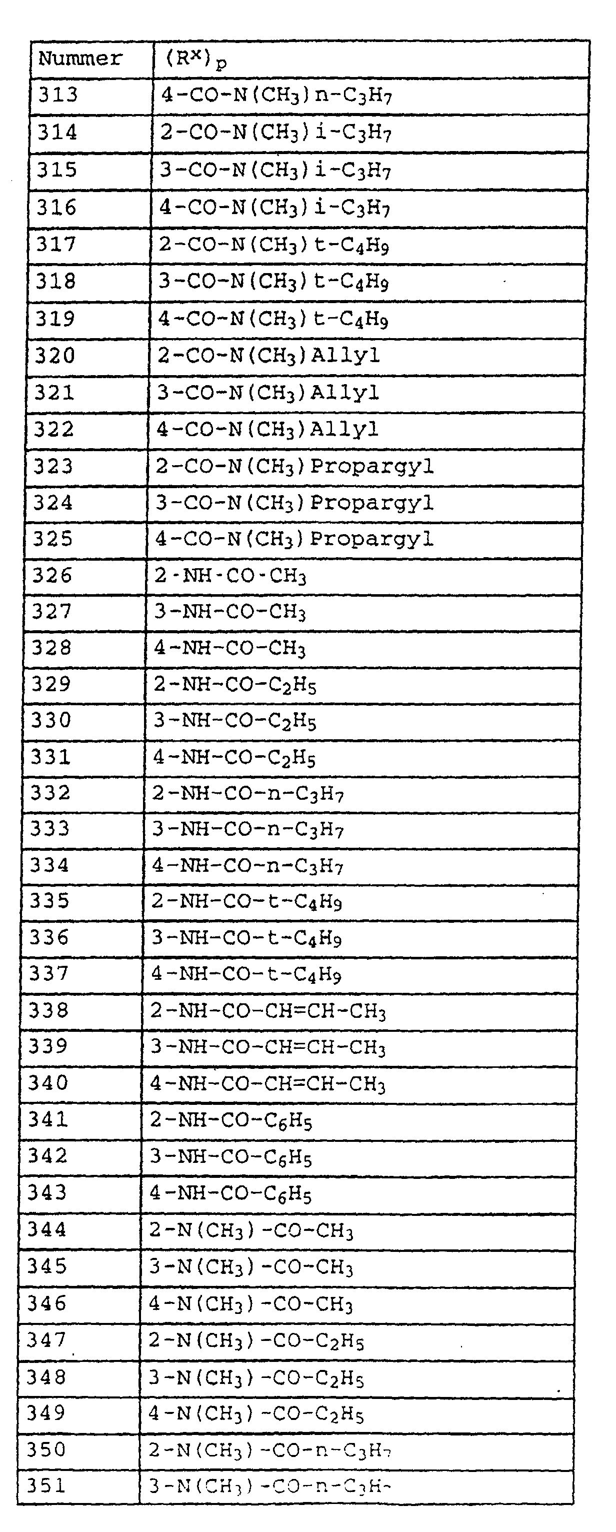

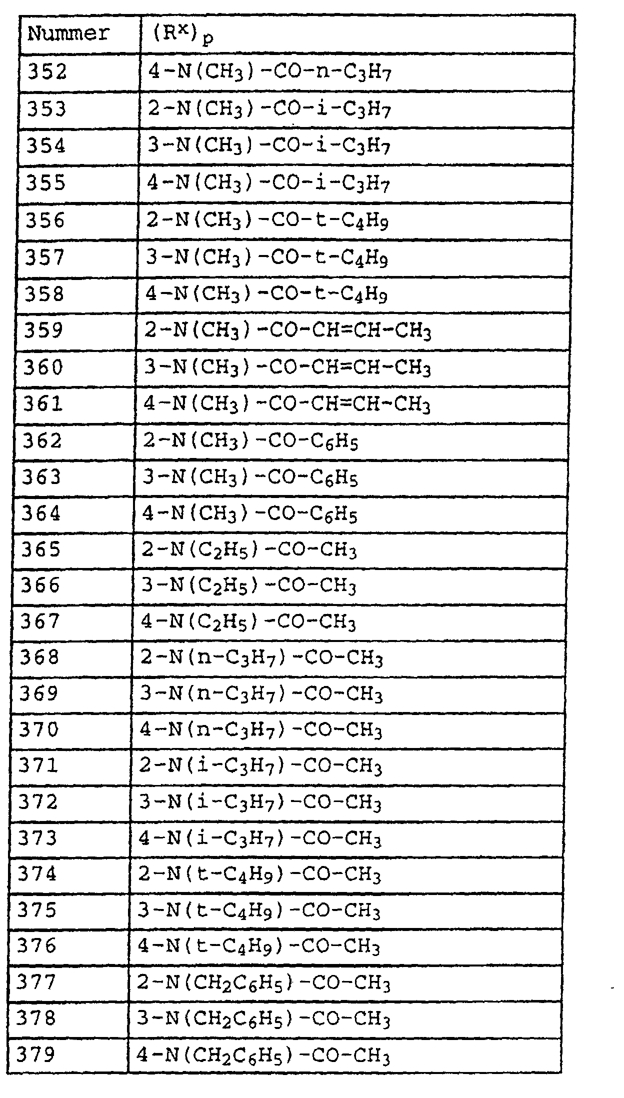

### Tabelle 2

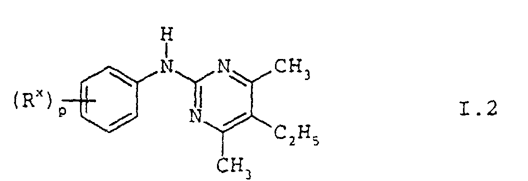

2-(N-Phenylamino)pyrimidine I.2-001 bis I.2-379 der allgemeinen Formel I.2, in welcher die Bedeutung von (R^{x})ₚ durch die Zeilen der Tabelle 1 gegeben ist.

### Tabelle 3

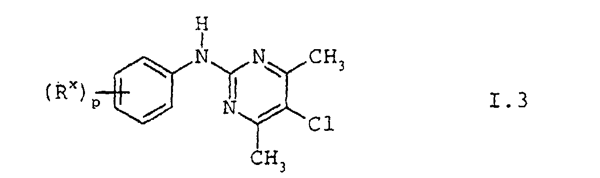

2-(N-Phenylamino)pyrimidine I.3-001 bis I.3-379 der allgemeinen Formel I.3, in welcher die Bedeutung von (R^{x})ₚ durch die Zeilen der Tabelle 1 gegeben ist.

### Tabelle 4

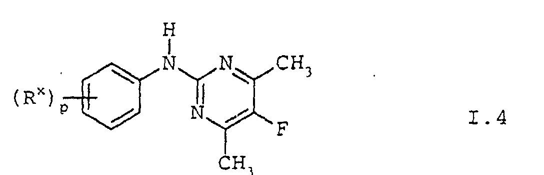

2-(N-Phenylamino)pyrimidine I.4-001 bis I.4-379 der allgemeinen Formel I.4, in welcher die Bedeutung von (R^{x})ₚ durch die Zeilen der Tabelle 1 gegeben ist.

### Tabelle 5

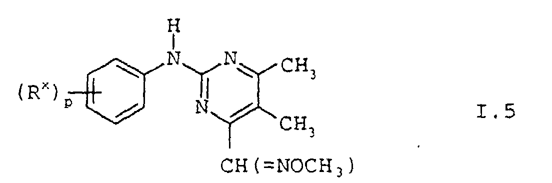

2-(N-Phenylamino)pyrimidine I.5-001 bis I.5-379 der allgemeinen Formel I.5, in welcher die Bedeutung von (R^{x})ₚ durch die Zeilen der Tabelle 1 gegeben ist.

### Tabelle 6

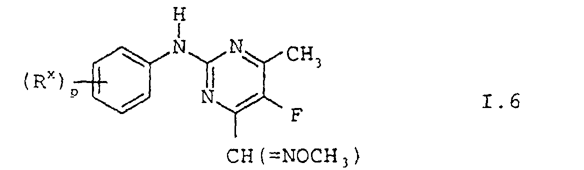

2-(N-Phenylamino)pyrimidine I.6-001 bis I.6-379 der allgemeinen Formel I.6, in welcher die Bedeutung von (R^{x})ₚ durch die Zeilen der Tabelle 1 gegeben ist.

### Tabelle 7

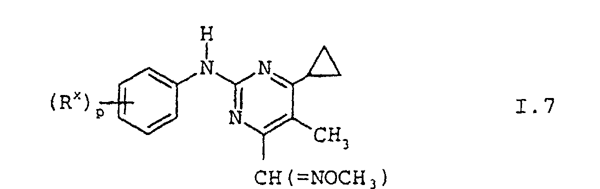

2-(N-Phenylamino)pyrimidine I.7-001 bis I.7-379 der allgemeinen Formel I.7, in welcher die Bedeutung von (R^{x})ₚ durch die Zeilen der Tabelle 1 gegeben ist.

### Tabelle 8

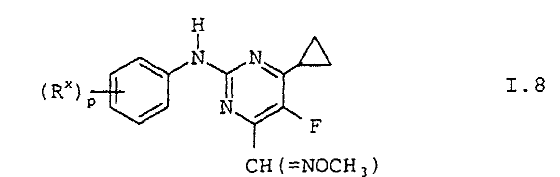

2-(N-Phenylamino)pyrimidine I.8-001 bis 1.8-379 der allgemeinen Formel I.8, in welcher die Bedeutung von (R^{x})ₚ durch die Zeilen der Tabelle 1 gegeben ist.

### Tabelle 9

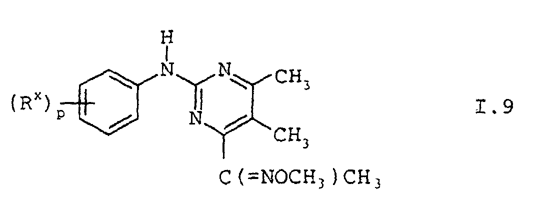

2-(N-Phenylamino)pyrimidine I.9-001 bis I.9-379 der allgemeinen Formel I.9, in welcher die Bedeutung von (R^{x})ₚ durch die Zeilen der Tabelle 1 gegeben ist.

### Tabelle 10

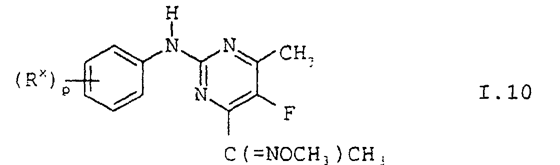

2-(N-Phenylamino)pyrimidine I.10-001 bis I.10-379 der allgemeinen Formel I.10, in welcher die Bedeutung von (R^{x})ₚ durch die Zeilen der Tabelle 1 gegeben ist.

### Tabelle 11

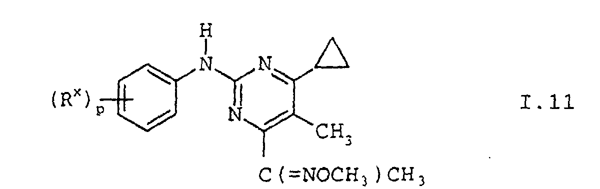

2-(N-Phenylamino)pyrimidine I.11-001 bis I.11-379 der allgemeinen Formel I.11, in welcher die Bedeutung von (R^{x})ₚ durch die Zeilen der Tabelle 1 gegeben ist.

### Tabelle 12

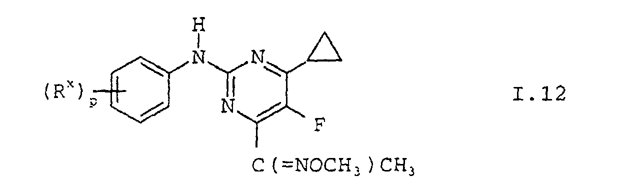

2-(N-Phenylamino)pyrimidine I.12-001 bis I.12-379 der allgemeinen Formel I.12, in welcher die Bedeutung von (R^{x})ₚ durch die Zeilen der Tabelle 1 gegeben ist.

### Tabelle 13

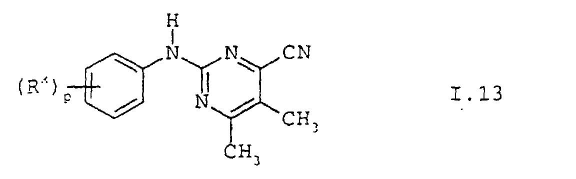

2-(N-Phenylamino)pyrimidine I.13-001 bis 1.13-379 der allgemeinen Formel I.13, in welcher die Bedeutung von (R^{x})ₚ durch die Zeilen der Tabelle 1 gegeben ist.

### Tabelle 14

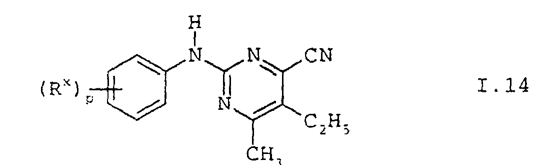

2-(N-Phenylamino)pyrimidine I.14-001 bis I.14-379 der allgemeinen Formel I.14, in welcher die Bedeutung von (R^{x})ₚ durch die Zeilen der Tabelle 1 gegeben ist.

### Tabelle 15

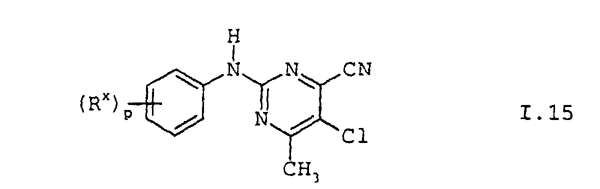

2-(N-Phenylamino)pyrimidine I.15-001 bis I.15-379 der allgemeinen Formel I.15, in welcher die Bedeutung von (R^{x})ₚ durch die Zeilen der Tabelle 1 gegeben ist.

### Tabelle 16

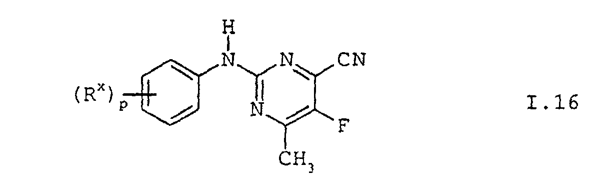

2-(N-Phenylamino)pyrimidine I.16-001 bis I.16-379 der allgemeinen Formel I.16, in welcher die Bedeutung von (R^{x})ₚ durch die Zeilen der Tabelle 1 gegeben ist.

### Tabelle 17

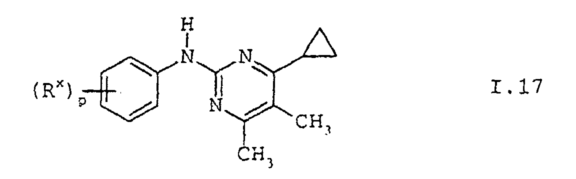

2-(N-Phenylamino)pyrimidine I.17-001 bis I.17-379 der allgemeinen Formel I.17, in welcher die Bedeutung von (R^{x})ₚ durch die Zeilen der Tabelle 1 gegeben ist.

### Tabelle 18

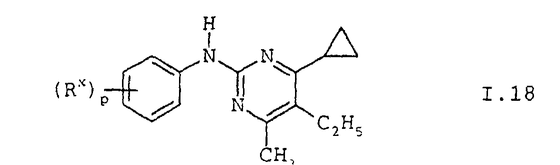

2-(N-Phenylamino)pyrimidine I.18-001 bis I.18-379 der allgemeinen Formel I.18, in welcher die Bedeutung von (R^{x})ₚ durch die Zeilen der Tabelle 1 gegeben ist.

### Tabelle 19

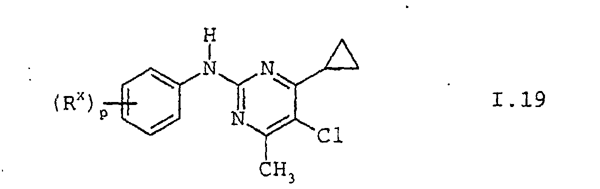

2-(N-Phenylamino)pyrimidine I.19-001 bis I.19-379 der allgemeinen Formel I.19, in welcher die Bedeutung von (R^{x})ₚ durch die Zeilen der Tabelle 1 gegeben ist.

### Tabelle 20

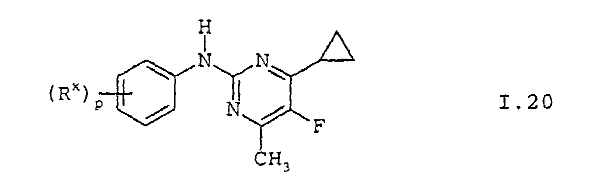

2-(N-Phenylamino)pyrimidine I.20-001 bis I.20-379 der allgemeinen Formel I.20, in welcher die Bedeutung von (R^{x})ₚ durch die Zeilen der Tabelle 1 gegeben ist.

### Tabelle 21

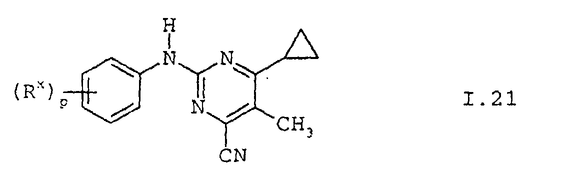

2-(N-Phenylamino)pyrimidine I.21-001 bis I.21-379 der allgemeinen Formel I.21, in welcher die Bedeutung von (R^{x})ₚ durch die Zeilen der Tabelle 1 gegeben ist.

### Tabelle 22

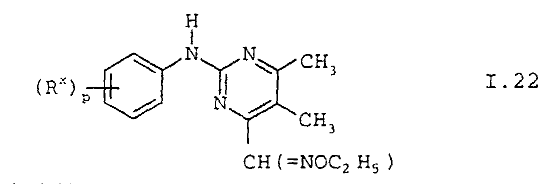

2-(N-Phenylamino)pyrimidine I.22-001 bis I.22-379 der allgemeinen Formel I.22, in welcher die Bedeutung von (R^{x})ₚ durch die Zeilen der Tabelle 1 gegeben ist.

### Tabelle 23

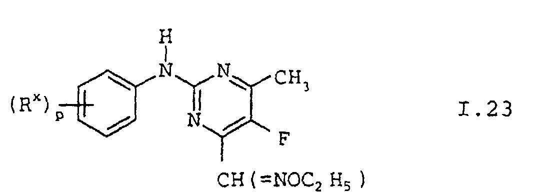

2-(N-Phenylamino)pyrimidine I.23-001 bis I.23-379 der allgemeinen Formel I.23, in welcher die Bedeutung von (R^{x})ₚ durch die Zeilen der Tabelle 1 gegeben ist.

### Tabelle 24

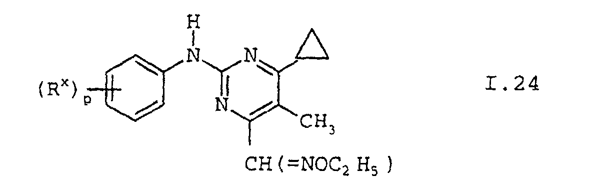

2-(N-Phenylamino)pyrimidine I.24-001 bis I.24-379 der allgemeinen Formel I.24, in welcher die Bedeutung von (R^{x})ₚ durch die Zeilen der Tabelle 1 gegeben ist.

### Tabelle 25

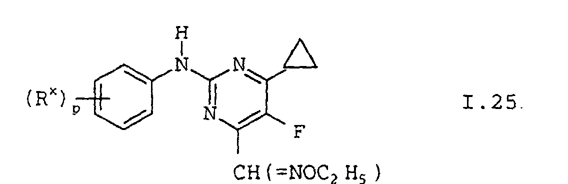

2-(N-Phenylamino)pyrimidine I.25-001 bis I.25-379 der allgemeinen Formel I.25, in welcher die Bedeutung von (R^{x})ₚ durch die Zeilen der Tabelle 1 gegeben ist.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten,* Deuteromyceten, *Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaftee, Zuckerrohr, Wein, Obst- und Zierpflanzen und Gemüsepflanzen
wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polycxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Konden sate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, 0,0-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio) -tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, M-[3-(p-tert.-Butylphenyl)-2-methylpropyl)-cis-2,6-dimethyl-morpholin, N-(3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(lH-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der AusgangsVerbindungen zur Gewinnung weiterer Verbindungen I benutzt. Die so erhaltenen Verbindungen sind in den anschließenden Tabellen mit physikalischen Angaben aufgeführt.

### 1. 4,6-Dimethyl-5-ethyl-2-(N-phenylamino)pyrimidin

Eine Mischung von 5,5 g (42 mmol) 3-Ethyl-acetylaceton und 6 g (30 mmol) Phenylguanidinium-hydrogencarbonat wurde ca. 3 Stunden auf 100°C erhitzt, wobei ein leichter Unterdruck (200 mbar) angelegt wurde. Anschließend wurde die Reaktionsmischung auf Raumtemperatur gekühlt und in Methylenchlorid aufgenommen. Die organische Phase wurde mit Wasser extrahiert, über Magnesiumsulfat getrocknet und eingeengt. Der Rückstand wurde säulenchromatographisch mit Cyclohexan/Essigester-Gemischen gereinigt. Man erhielt 2,1 g (31%) der Titelverbindung als hellen Festkörper.
¹H-NMR (CDCl₃; δ in ppm) : 7,65 (d,2H); 7,35 (s,breit,1H) ; 7,3 (t,2H) ; 6,95 (t,1H) ; 2,55 (q,2H) ; 2,4 (s,6H) ; 1,1 (t,3H).

### 2. 5,6-Dimethyl-4-(N-methoxyimino)methyl-2-(N-phenylamino)pyrimidin

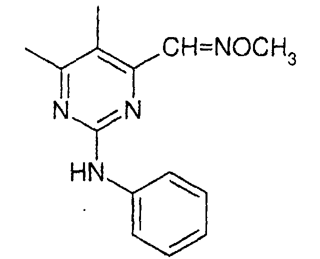

### a) 1,1-Diethoxy-3-methyl-pentan-2,4-dion

Eine Lösung von 43 g (0,22 mol) 1,1-Diethoxy-pentan-2,4-dion in 400 ml Methylenchlorid wurde bei 0°C mit 60 g (0,38 mol) Methyl-iodid und 36 g (0,23 mol) Diazabicyclo-undecen (DBU) versetzt.
Anschließend wurde ca. 3 Stunden bei Raumtemperatur gerührt. Dann gab man zusätzlich 20 g (0,13 mol) DBU hinzu und rührte über Nacht bei Raumtemperatur.
Dann wurde die Reaktionsmischung mit Wasser, verdünnter Salzsäure (pH = 1) und Wasser extrahiert, über Magnesiumsulfat getrocknet und eingeengt. Als Rückstand erhielt man 44 g eines gelben Öls, das laut GC und ¹H-NMR ca. 50 % der Titelverbindung, 40 % 1,1-Diethoxy-3,3-dimethyl-pentan-2,4-dion sowie ca. 3 % 1,1-Diethoxy-pentan-2,4-dion enthielt.
Das so erhaltene Rohprodukt wurde ohne weitere Reinigung in die nächste Reaktion eingesetzt.
¹H-NMR (CDCl₃, δ in ppm) :
4,65 (s, 1H) ; 4,05 (q, 1H) ; 3,7 (m, 4H); 2,25 (s, 3H) ; 1,2 (m, 9H)

### b) 5,6-Dimethyl-4-diethoxymethyl-2-(phenylamino)pyrimidin

Eine Mischung von 44 g (Reinheit ca. 50 %ig, ca. 0,11 mol) 1,1-Diethoxy-3-methyl-pentan-2,4-dion (Rohprodukt, Beispiel 2a) und 35 g (0,175 mol) Phenylguanidinium-hydrogencarbonat in 300 ml Ethanol wurde ca. 3 Stunden auf 70-80°C erhitzt. Anschließend wurde die Reaktionsmischung eingeengt und der Rückstand wurde mit Methyl-t-butylether über Kieselgel abgesaugt. Das Lösungsmittel wurde abgedampft. Schwerer flüchtige Bestandteile wurden bei einem Druck von 0,3 mbar und einer Badtemperatur von 150°C abdestilliert. Als Rückstand erhielt man 38 g (Ausbeute quantitativ) der Titelverbindung als gelbes Öl.
¹H-NMR (CDCl₃, δ in ppm) :
7,7 (d, 2H); 7,3 (t, 2H); 7,1 (s, breit, 1H) ; 7,0 (t, 1H) ; 5,35 (s, 1H) ; 3,75 (m, 2H) ; 3,6 (m, 2H) ; 2,45 (s, 3H) ; 2,25 (s, 3H); 1,25 (t, 6H)

### c) 5,6-Dimethyl-4-formyl-2-(phenylamino)pyrimidin

Eine Mischung von 38 g (0,126 mol) 5,6-Dimethyl-4-diethoxymethyl-2-(phenylamino)pyrimidin (Beispiel 2b) und 50 ml konz. Salzsäure in 400 ml Wasser wurde ca. 3 Stunden auf 60°C erhitzt.
Anschließend wurde die Reaktionsmischung abgekühlt, wobei ein Festkörper ausfiel. Die Reaktionsmischung wurde mit verdünnter Natronlauge auf pH = 6 gestellt und der ausgefallene Festkörper wurde abgesaugt. Sodann wurde der Festkörper mit Aceton gewaschen und bei einem Druck von ca. 100 mbar und einer Temperatur von 50°C 48 Stunden getrocknet. Man erhielt 29 g (Ausbeute quantitativ) der Titelverbindung als gelben Fest-körper.
¹H-NMR (DMSO-d₆, δ in ppm) : 9,95 (s, 1H) ; 9,75 (s, breit, 1H) ; 7,8 (d, 2H) ; 7,3 (t, 2H) ; 6,95 (t, 1H) ; 2,5, (s, 3H) ; 2,3 (s, 3H)

### d) 5,6-Dimethyl-4-(N-methoxyimino)methyl-2-(phenylamino)pyrimidin

Eine Mischung von 1,1 g (5 mmol) 5,6-Dimethyl-4-formyl-2-(phenylamino)pyrimidin (Beispiel 2c) und 0,5 g (6 mmol) O-Methylhydroxylamin-hydrochlorid in 20 ml Methanol wurde ca. 1 Stunde zum Rückfluß erhitzt.
Anschließendd wurde die Reaktionsmischung am Rotationsverdampfer eingeengt. Der Rückstand wurde in Methylenchlorid aufgenommen und die organische Phase wurde mit NaHCO₃-Lsg. extrahiert. Das Lösungsmittel wurde abgedampft und der Rückstand wurde säulenchromatographisch mit Cyclohexan/Methylt-butylether-Gemischen gereinigt. Man erhielt 0,8 g (3,1 mmol, 63 %) der Titelverbindung als gelben Festkörper (Fp = 85-88°C).
¹H-NMR (CDCl₃, δ in ppm) :
8,2 (s, 1H) ; 7,65 (d, 2H); 7,3 (t, 2H) ; 7,1 (s, breit, 1H) ; 7,0 (t, 1H) ; 4,05 (s, 3H) ; 2,5 (s, 3H); 2,3 (s, 3H)

### 3. 4-Cyano-5,6-Dimethyl-2-(phenylamino)pyrimidin

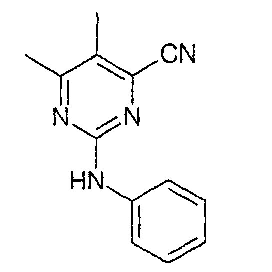

Eine Mischung von 2,5 g (11 mmol) 5,6-Dimethyl-4-formyl-2-phenylaminopyrimidin (Beispiel 2c) und 1,2 g (17 mmol) Hydroxylamin-hydrochlorid in 10 ml Pyridin wurde ca. 1,5 Stunden auf 50°C erwärmt.
Anschließend tropfte man 3,5 ml (37 mmol) Acetanhydrid hinzu und rührte die Reaktionsmischung ca. 1,5 Stunde bei 90°C. Dann wurde die Reaktionsmischung am Rotationsverdampfer eingeengt. Der Rückstand wurde mit Wasser verdünnt und mit verdünnter Natronlauge auf pH 7-8 gestellt. Die wässrige Phase wurde mit Essigester und Methylenchlorid extrahiert.

Anschließend wurden die vereinigten organischen Phasen über Kieselgel abgesaugt, wobei mit Methylenchlorid/Essigester 2:1 nachgewaschen wurde. Dann wurde die organische Phase eingeengt. Der Rückstand kristallisierte und wurde mit Diisopropylether ausgerührt.
Man erhielt 1,9 g (75 %) der Titelverbindung als hellen Festkörper (Fp = 181-184°C).
¹H-NMR (CDCl₃, δ in ppm) :
7,6 (d, 2H); 7,35 (t, 2H) ; 7,15 (s, breit, 1H) ; 7,05 (t, 1H) ; 2,5 (s, 3H) ; 2,35 (s, 3H)

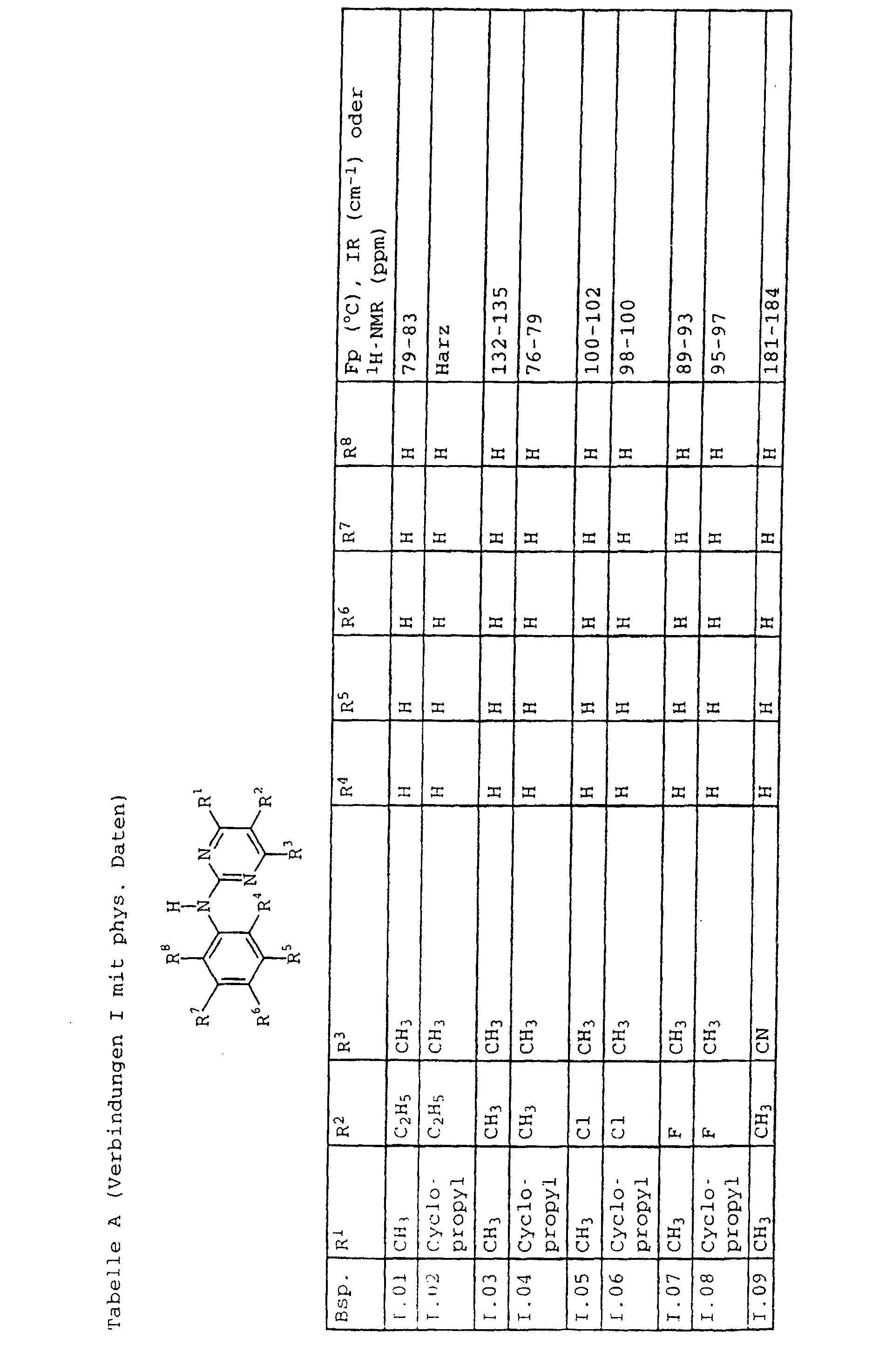

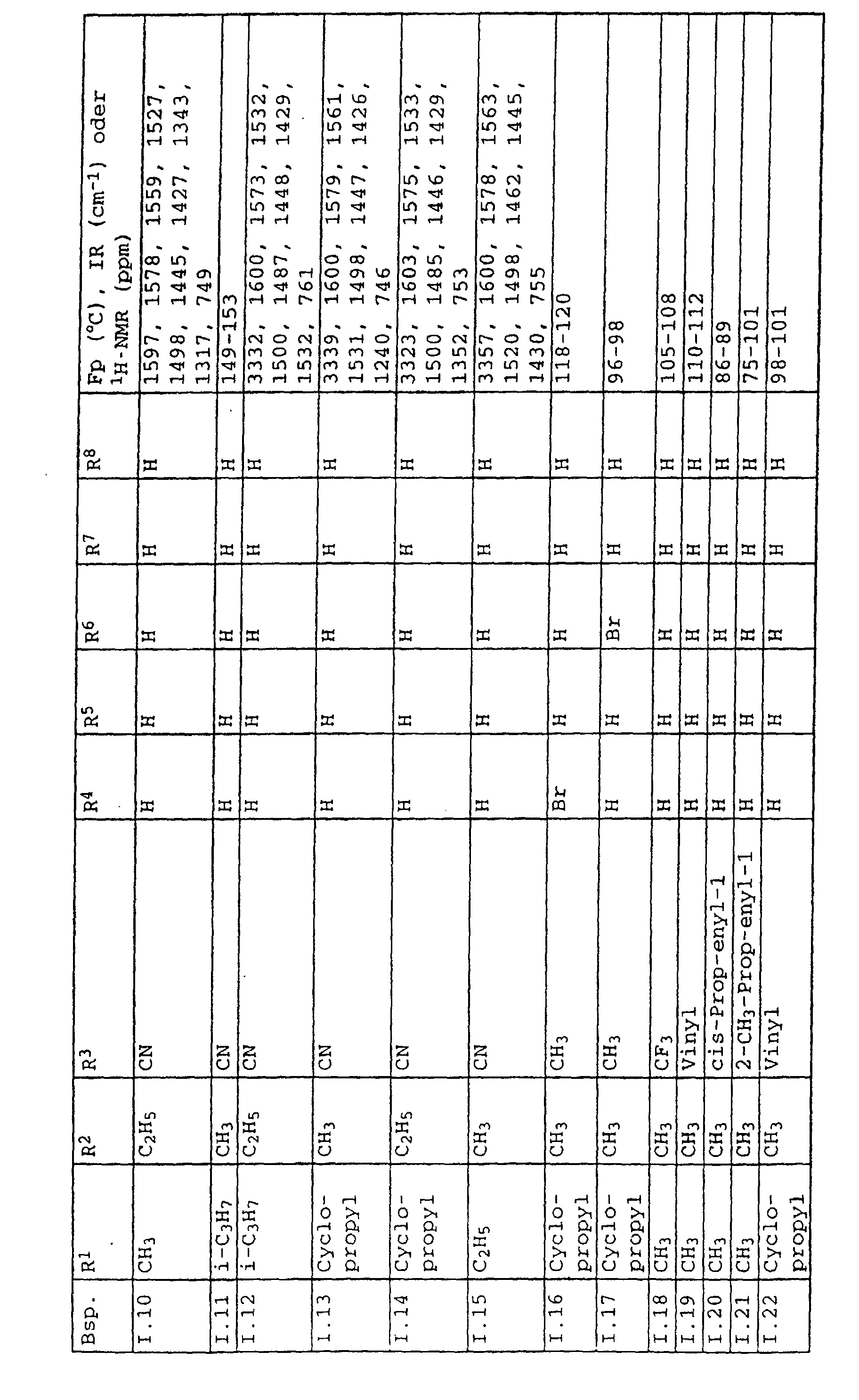

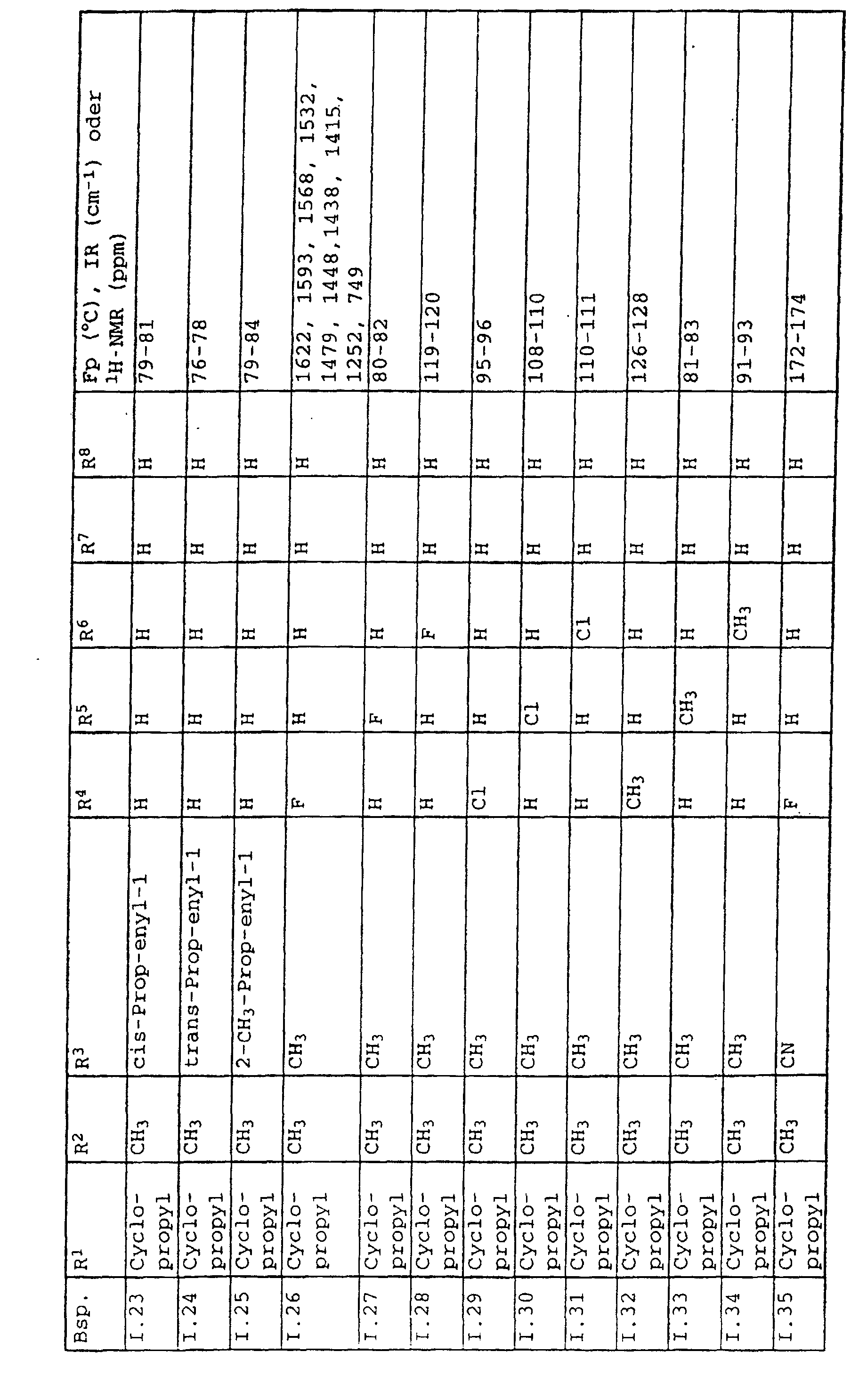

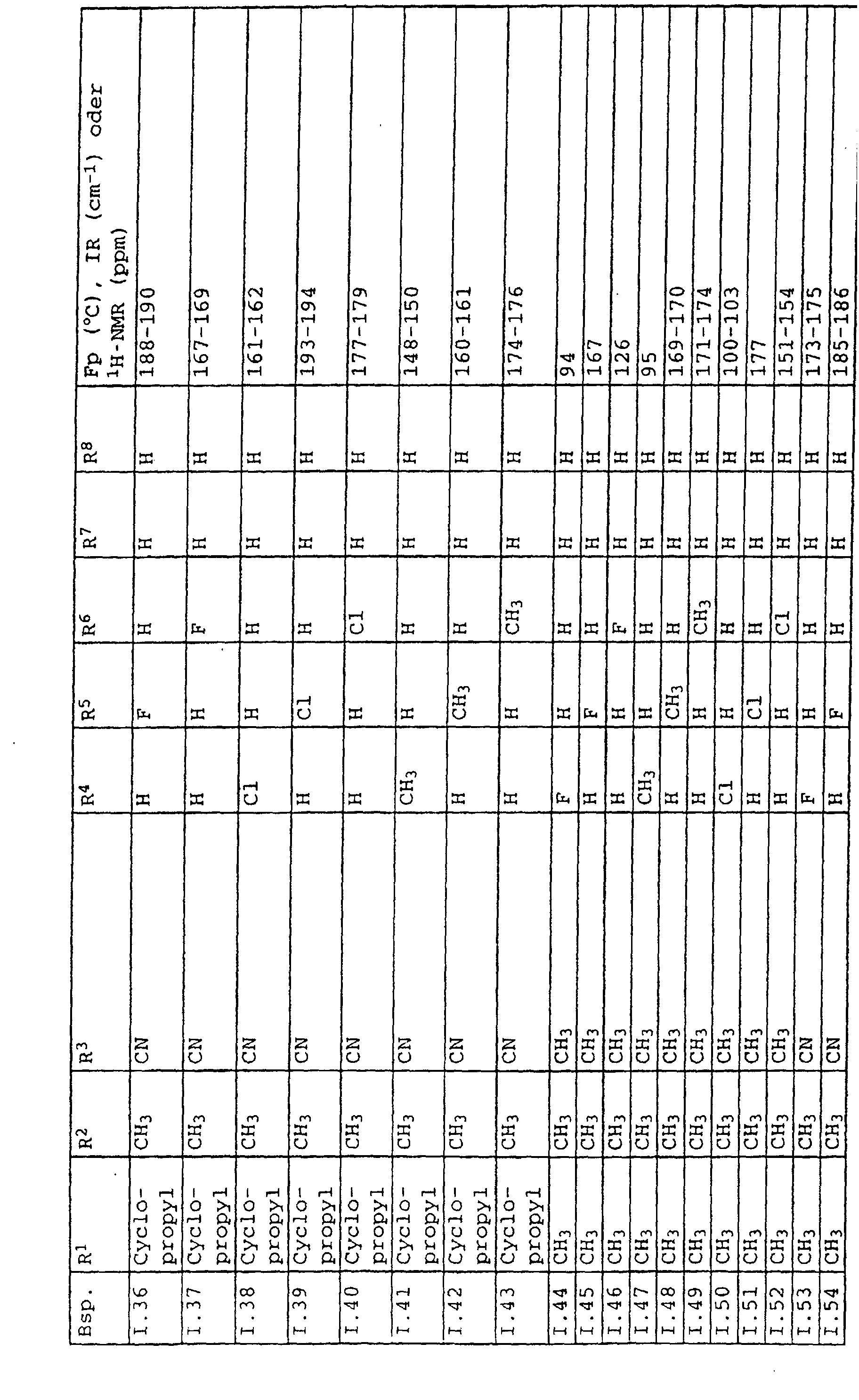

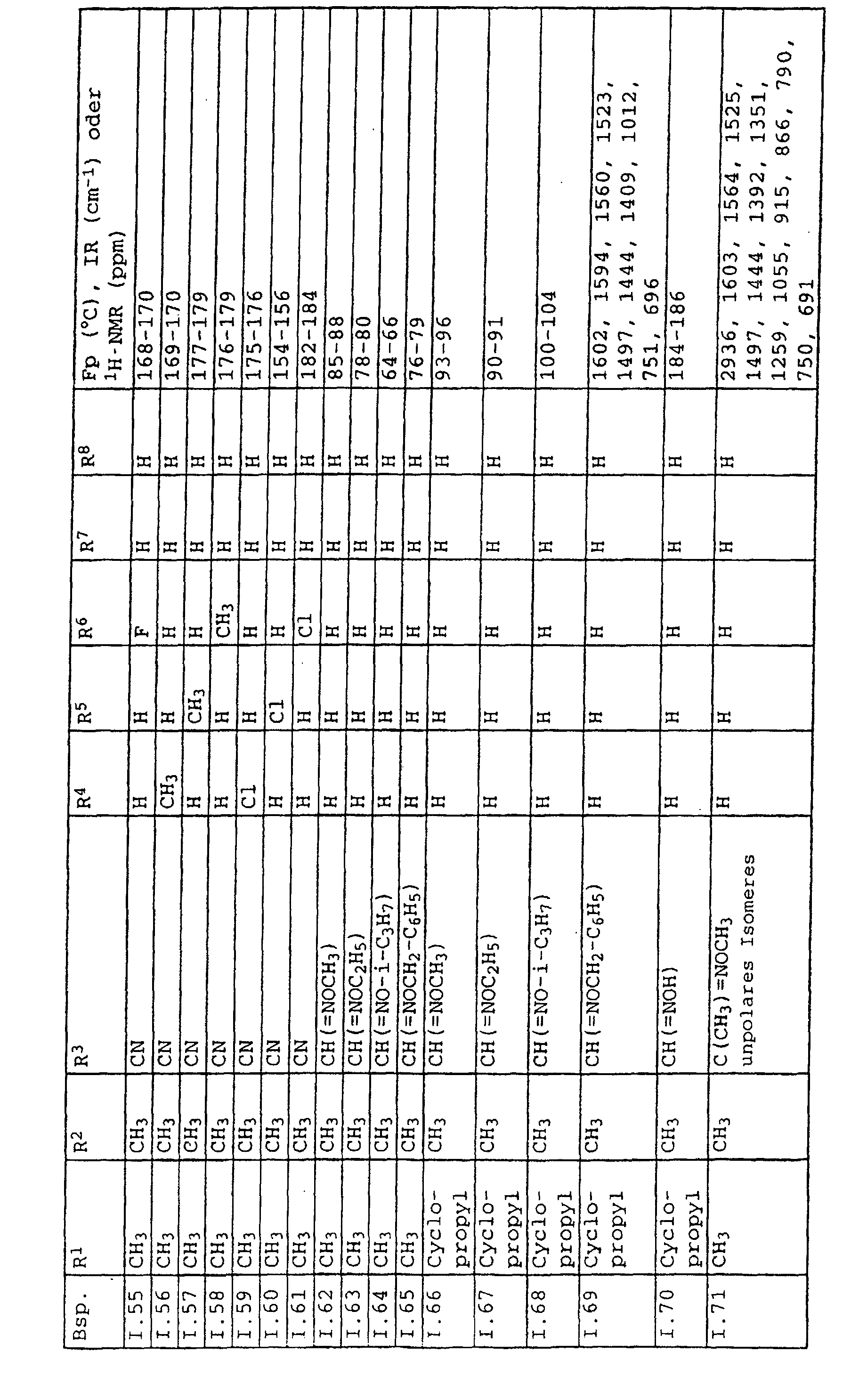

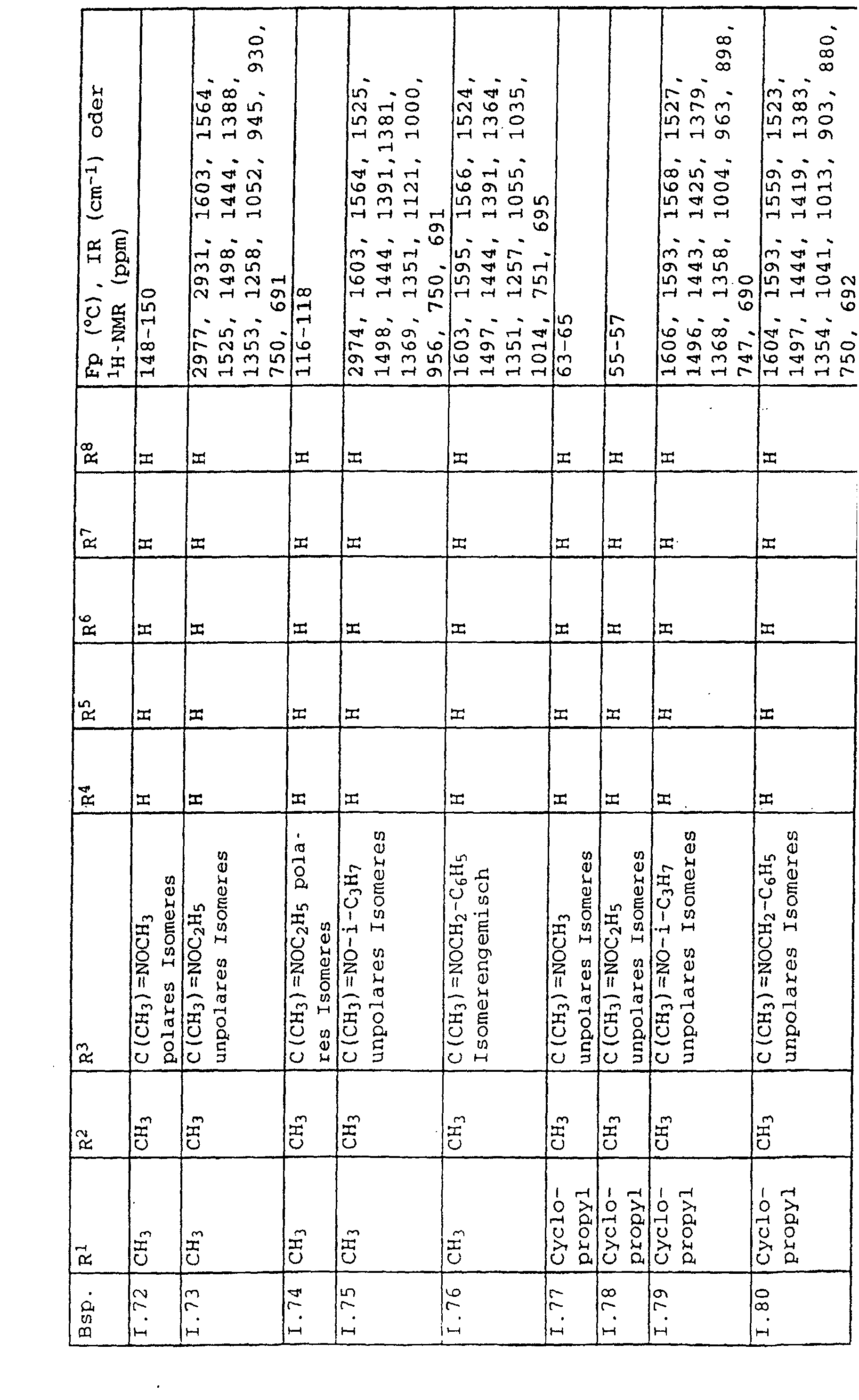

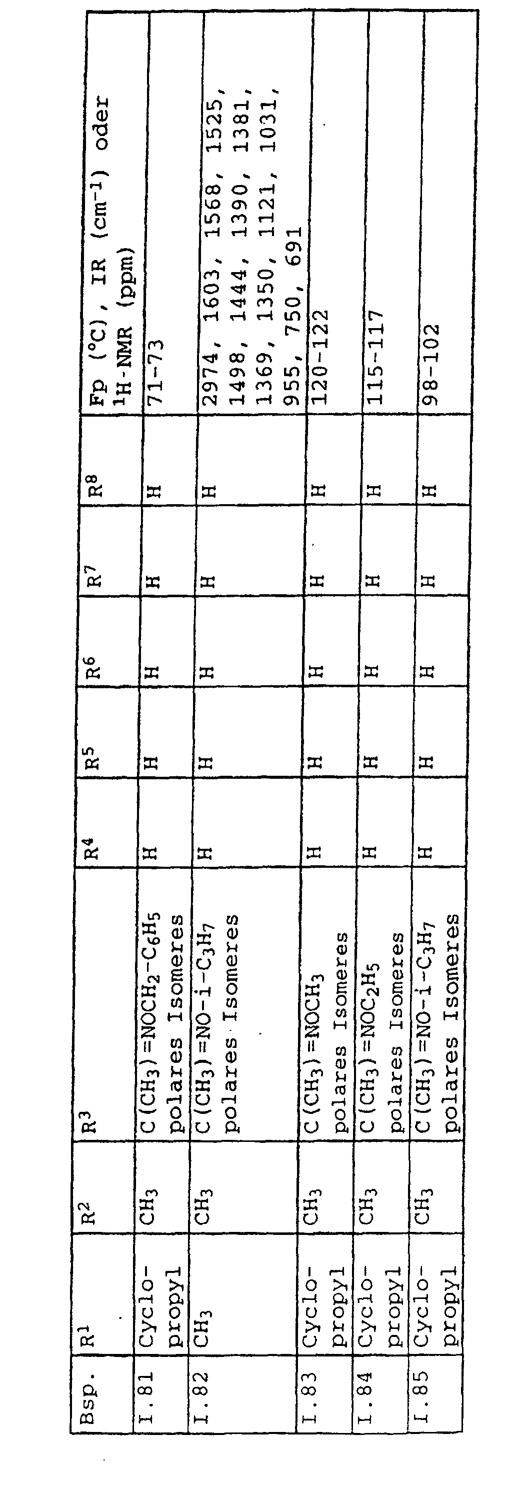

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Als Vergleichsverbindungen dienten:
a) der aus EP-A 457 726 bekannte Wirkstoff A (Tabelle 1, Nr. 1.1) 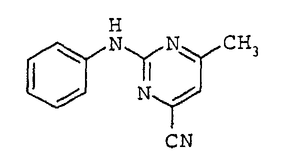
b) Wirkstoff B (fällt unter die Ansprüche der EP-A 270 111) 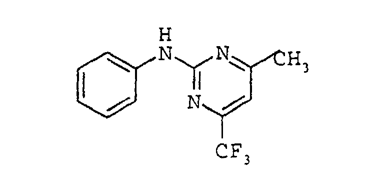
c) der aus JP 03-271 278 bekannte Wirkstoff C (Tabelle 1, Nr. 2)

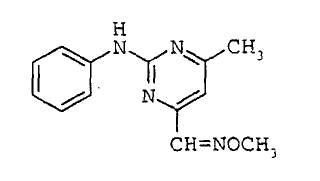

### Vergleichsversuch 1 - Wirksamkeit gegen Plasmopara viticola

Blätter von Topfreben der Sorte "Müller-Thurgau" wurden mit 500 ppm wäßriger Wirkstoffaufbereitung, die mit einer Stammlösung aus 10 % Wirkstoff, 63 % Cyclohexanon und 27 % Emulgiermittel angesetzt wurde, bis zur Troofnäße besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Versuch zeigten die mit dem erfindungsgemäßen Wirkstoff I.9 behandelten Pflanzen einen Befall von 5 % während die mit dem bekannten Wirkstoff A behandelten Pflanzen zu 50 % und die unbehanditen Pflanzen zu 85 % befallen waren.

### Vergleichsversuch 2 - Kurative Wirksamkeit gegen Puccinia recondita an Weizen (Weizenbraunrost)

Blätter von in Töpfen gewachsenen Weizensämlingen der Sorte "Kanzler" wurden mit Sporen des Braunrostes (*Puccinia recondita*) bestäubt. Danach wurden die Töpfe für 24 Stunden in eine Kammer mit hoher Luftfeuchtigkeit (90 bis 95 %) und 20 bis 22° C gestellt. Während dieser Zeit keimten die Sporen aus und die Keimschläuche drangen in das Blattgewebe ein. Die infizierten Pflanzen wurden am nächsten Tag mit 250 ppm einer wäßrigen Wirkstoffaufbereitung, die aus einer Stammlösung bestehend aus 10 % Wirkstoff, 63 % Cyclohexanon und 27 % Emulgiermittel angesetzt worden war, tropfnaß besprüht. Nach dem Antrocknen des Spritzbelages wurden die Versuchspflanzen im Gewächshaus bei Temperaturen zwischen 20 und 22° C und 65 bis 70 % relativer Luftfeuchte für 7 Tage kultiviert. Dann wurde das Ausmaß der Rostpilzentwicklung auf den Blättern ermittelt.

In diesem Versuch zeigten die mit dem erfindungsgemäßen Wirkstoff I.9 behandelten Pflanzen einen Befall von 30 % während die mit dem bekannten Wirkstoff A behandelten Pflanzen und die unbehandlten Pflanzen zu 100 % befallen waren.

### Vergleichsversuch 3 - Wirksamkeit gegen Pyricularia oryzae (protektiv)

Blätter von in Töpfen gewachsenen Reiskeimlingen der Sorte "Tai-Nong 67" wurden mit 250 ppm wäßriger Wirkstoffaufbereitung, die mit einer Stammlösung aus 10 % Wirkstoff, 63 % Cyclohexanon und 27 % Emulviermittel angesetzt wurde, bis zur Tropfnässe besprüht. Am folgenden Tag wurden die Pflanzen mit einer wäßrigen Sporensuspension von *Pyricularia oryzae* inokuliert. Anschließend wurden die Versuchspflanzen in Klimakammern bei 22 - 24° C und 95 - 99 % relativer Luftfeuchtigkeit für 6 Tage aufgestellt. Dann wurde das Ausmaß der Befallsentwicklung auf den Blättern visuell ermittelt.

In diesem Versuch zeigten die mit dem erfindungsgemäßen Wirkstoff I.18 behandelten Pflanzen einen Befall von 15 % während die mit dem bekannten Wirkstoff B behandelten Pflanzen zu 80 % und die unbehandlten Pflanzen zu 90 % befallen waren.

### Vergleichsversuch 4 - Wirksamkeit gegen Plasmopara vikicola

Blätter von Topfreben der Sorte "Müller-Thurgau" wurden mit 250 ppm wäßriger Wirkstoffaufbereitung, die mit einer Stammlösung aus 10 % Wirkstoff, 63 % Cyclohexanon und 27 % Emulgiermittel angesetzt wurde, bis zur Tropfnäße besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoo- sporenaufschwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Versuch zeigten die mit dem erfindungsgemäßen Wirkstoff I.62 behandelten Pflanzen einen Befall von 10 % während die mit dem bekannten Wirkstoff C behandelten Pflanzen zu 60 % und die unbehandlten Pflanzen zu 85 % befallen waren.

## Patentansprüche

1. Verwendung von 2-(N-Phenylamino)pyrimidinen der Formel I, 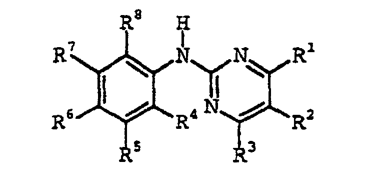 in der die Substituenten die folgende Bedeutung haben:
R¹, R³ unabhängig voneinander Cyano, C₁-C₈-Alkyl, C₂-C₆-Alkenyl, C₂-C₆-Alkinyl, wobei die Reste Alkyl, Alkenyl und Alkinyl durch Cyano, Halogen, C₁-C₄-Alkoxy oder C₁-C₄-Alkoxycarbonyl substituiert sein können, C₃-C₈-Cycloalkyl oder eine Gruppe C(=NOR^{x})R^{y};
R^{x} Wasserstoff, C₁-C₈-Alkyl, C₂-C₆-Alkenyl, C₂-C₆-Alkinyl, wobei die Reste Alkyl, Alkenyl und Alkinyl durch Cyano, Halogen, C₁-C₄-Alkoxy oder Phenyl substituiert sein können;
R^{y} Wasserstoff, C₁-C₈-Alkyl, C₂-C₆-Alkenyl, C₂-C₆-Alkinyl, wobei die Reste Alkyl, Alkenyl und Alkinyl durch Cyano, Halogen oder C₁-C₄-Alkoxy substituiert sein können;
R² Halogen, C₁-C₈-Alkyl, C₂-C₆-Alkenyl, C₂-C₆-Alkinyl, wobei die Reste Alkyl, Alkenyl und Alkinyl durch Cyano, Halogen, C₁-C₄-Alkoxy oder C₁-C₄-Alkoxycarbonyl substituiert sein können;
R⁴ bis R⁸ unabhangig voneinander Wasserstoff, Cyano, Halogen, R^{a}, R^{a}O, R^{a}S(O)ₘ, R^{a}O-(C=O), R^{a}(C=O), R^{a}R^{b}N-(C=O), R^{a}HN- (C=O), H₂N- (C=O), R^{a}- (C=O) -NH, R^{a}- (C=O) -NR^{b} oder R^{a}O-N=C(R^{b}) ;
R^{a}, R^{b} C₁-C₄-Alkyl, C₃-C₆-Alkenyl oder C₃-C₆-Alkinyl, welche jeweils durch Cyano, Halogen, C₁-C₄-Alkoxy, C₁-C₄-Alkoxycarbonyl oder Phenyl substituiert sein können oder
Phenyl, welches ein bis drei Substituenten ausgewählt aus der Gruppe: Halogen und C₁-C₄-Alkyl tragen kann;
m 0, 1 oder 2;
als Fungizide.

2. 2-(N-Phenylamino)pyrimidine der Formel I, in der
R¹, R³ unabhängig voneinander Cyano, C₁-C₈-Alkyl, C₂-C₆-Alkenyl, C₂-C₆-Alkinyl oder C₃-C₈-cycloalkyl oder eine Gruppe C(=NOR^{x})R^{y};
R^{x} Wasserstoff oder C₁-C₈-Alkyl;
R^{y} Wasserstoff oder C₁-C₈-Alkyl;
R² Halogen, C₁-C₈-Alkyl, C₂-C₆-Alkenyl, C₂-C₆-Alkinyl oder C₁-C₂-Haloalkyl;
R⁴, R⁶, R⁸ unabhängig voneinander Wasserstoff, Cyano, Halogen, R^{a}, R^{a}O, R^{a}S(O)ₘ, R^{a}O-(C=O), R^{a} (C=O), R^{a}R^{b}N-(C=O), R^{a}HN-(C=O), H₂N-(C=O), R^{a}-(C=O)-NH, R^{a}-(C=O)-NR^{b} oder R^{a}O-N=C(R^{b});
R⁵, R⁷ unabhängig voneinander Wasserstoff, Cyano, R^{a}, R^{a}O, R^{a}S(O)ₘ, R^{a}O-(C=O), R^{a}(C=O), R^{a}R^{b}N-(C=O), R^{a}HN- (C=O), H₂N-(C=O), R^{a}-(C=O)-NH, R^{a}-(C=O)-NR^{b} oder R^{a}O-N=C(R^{b}) ;
R^{a}, R^{b} C₁-C₄-Alkyl, C₃-C₆-Alkenyl oder C₃-C₆-Alkinyl, welche jeweils durch Cyano, Halogen, C₁-C₄-Alkoxy, C₁-C₄-Alkaxycarbonyl oder Phenyl substituiert sein können oder
Phenyl, welches ein bis drei Substituanten ausgewählt aus der Gruppe; Halogen und C₁-C₄-Alkyl tragen kann, und
m 0, 1 oder 2 bedeuten.

3. 2-(N-Phenylamino)pyrimidine der Formel I gemäß Anspruch 2, wobei
R¹ Methyl oder Cyclopropyl bedeutet.

4. 2-(N-Phenylamino)pyrimidine der Formel I gemäß den Ansprüchen 2 und 3, wobei
R⁴ bis R⁸ Wasserstoff bedeuten.

5. 2-(N-Phenylamino)pyrimidine der Formel I qemäß den Ansprüchen 2-4, wobei
R² Halogen oder Methyl bedeutet.

6. 2-(N-Phenylamino)pyrimidine der Formel I gemäß den Ansprüchen 2-5, wobei
R³ CH(=NOCH₃), CH(=NOC₂H₅), C(=NOCH₃)CH₃, C(=NOC₂H₅)CH₃, Cyano oder Methyl bedeutet.

7. 2-(N-Phenylamino)pyrimidine der Formel I, in der
R¹ Methyl oder Cyclopropyl;
R² Halogen oder Methyl;
R³ Trifluormethyl;
R⁴, R⁸ Wasserstoff;
R⁵, R⁷ unabhängig voneinander Wasserstoff, Cyano, C₁-C₂-Haloalkoxy, R^{a}, R^{a}O, R^{a}S(O)ₘ, R^{a}O-(C=O), R^{a}(C=O), R^{a}R^{b}N- (C=O), R^{a}HN-(C=O), H₂N-(C=O), R^{a}-(C=O)-NH, R^{a}-(C=O)-NR^{b} oder R^{a}O-N=C(R^{b}) ;
R⁶ unabhängig voneinander Wasserstoff, Cyano, Halogen, C₁-C₂-Haloalkyl, C₁-C₂-Haloalkoxy, R^{a}, R^{a}O, R^{a}S(O)ₘ, R^{a}O-(C=O), R^{a}(C=O), R^{a}R^{b}N- (C=O), R^{a}HN-(CO),H₂N-(C=O), R^{a}-(C=O)-NH, R^{a}-(C=O)-NR^{b} oder R^{a}O-N=C(R^{b}) ;
R^{a}, R^{b} C₁-C₄-Alkyl, C₃-C₆-Alkanyl oder C₃-C₆-Alkinyl, welche jeweils durch Cyano, C₁-C₄-Alkoxy, C₁-C₄-Alkoxycarbonyl oder Phenyl substituiert sein können oder Phenyl, welches ein bis drei Substituenten ausgewäh aus der Gruppe: Halogen und C₁-C₄-Alkyl tragen kann,
bedeuten.

## Claims

1. The use of 2-(N-phenylamino)pyrimidines of the formula I, 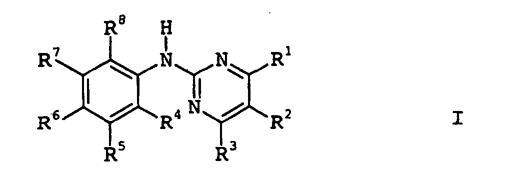 where:
R¹, R³ independently of one another are cyano, C₁-C₈-alkyl, C₂-C₆-alkenyl, C₂-C₆-alkynyl, where the radicals alkyl, alkenyl and alkynyl may be substituted by cyano, halogen, C₁-C₄-alkoxy or C₁-C₄-alkoxycarbonyl, or are C₃-C₈-cycloalkyl or a group C(=NOR^{x})R^{y} ;
R^{x} is hydrogen, C₁-C₈-alkyl, C₂-C₆-alkenyl, C₂-C₆-alkynyl, where the radicals alkyl, alkenyl and alkynyl may be substituted by cyano, halogen, C₁-C₄-alkoxy or phenyl;
R^{y} is hydrogen, C₁-C₈-alkyl, C₂-C₆-alkenyl, C₂-C₆-alkynyl, where the radicals alkyl, alkenyl and alkynyl may be substituted by cyano, halogen or C₁-C₄-alkoxy;
R² is halogen, C₁-C₈-alkyl, C₂-C₆-alkenyl, C₂-C₆-alkynyl, where the radicals alkyl, alkenyl and alkynyl may be substituted by cyano, halogen, C₁-C₄-alkoxy or C₁-C₄-alkoxycarbonyl; or
R⁴ to R⁸ independently of one another are hydrogen, cyano, halogen, R³, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} or R^{a}O-N=C(R^{b}) ;
R^{a}, R^{b} are C₁-C₄-alkyl, C₃-C₆-alkenyl or C₃-C₆-alkynyl, each of which may be substituted by cyano, halogen, C₁-C₄-alkoxy, C₁-C₄-alkoxycarbonyl or phenyl, or are phenyl which may carry one to three substituents selected from the group consisting of halogen and C₁-C₄-alkyl;
m is 0, 1 or 2;
as fungicides.

2. A 2-(N-phenylamino)pyrimidine of the formula I in which
R¹, R³ independently of one another are cyano, C₁-C₈-alkyl, C₂-C₆-alkenyl, C₂-C₆-alkynyl or C₃-C₈-cycloalkyl or a group C(=NOR^{x})R^{y};
R^{x} is hydrogen or C₁-C₈-alkyl;
R^{y} is hydrogen or C₁-C₈-alkyl;
R² is halogen, C₁-C₈-alkyl, C₂-C₆-alkenyl, C₂-C₆-alkynyl or C₁-C₂-haloalkyl;
R⁴,R⁶,R⁸ independently of one another are hydrogen, cyano, halogen, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} or R^{a}O-N=C(R^{b});
R⁵, R⁷ independently of one another are hydrogen, cyano, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} or R^{a}O-N=C(R^{b});
R^{a}, R^{b} are C₁-C₄-alkyl, C₃-C₆-alkenyl or C₃-C₆-alkynyl, each of which may be substituted by cyano, halogen, C₁-C₄-alkoxy, C₁-C₄-alkoxycarbonyl or phenyl, or are phenyl which may carry one to three substituents selected from the group consisting of halogen and C₁-C₄-alkyl, and
m is 0, 1 or 2.

3. A 2-(N-phenylamino)pyrimidine of the formula I as claimed in claim 2, wherein
R¹ is methyl or cyclopropyl.

4. A 2-(N-phenylamino)pyrimidine of the formula I as claimed in claim 2 or 3, wherein
R⁴ to R⁸ are hydrogen.

5. A 2-(N-phenylamino)pyrimidine of the formula I as claimed in any of claims 2 to 4, wherein
R² is halogen or methyl.

6. A 2-(N-phenylamino)pyrimidine of the formula I as claimed in any of claims 2 to 5, wherein
R³ is CH(=NOCH₃), CH(=NOC₂H₅), C(=NOCH₃)CH₃, C(=NOC₂H₅)CH₃, cyano or methyl.

7. A 2-(N-phenylamino)pyrimidine of the formula I in which
R¹ is methyl or cyclopropyl;
R² is halogen or methyl;
R³ is trifluoromethyl;
R⁴, R⁸ are hydrogen;
R⁵, R⁷ independently of one another are hydrogen, cyano, C₁-C₂-haloalkoxy, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} or R^{a}O-N=C(R^{b}) ;
R⁶ is hydrogen, cyano, halogen, C₁-C₂-haloalkyl, C₁-C₂-haloalkoxy, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} or R^{a}O-N=C(R^{b}) ;
R^{a}, R^{b} are C₁-C₄-alkyl, C₃-C₆-alkenyl or C₃-C₆-alkynyl, each of which may be substituted by cyano, C₁-C₄-alkoxy, C₁-C₄-alkoxycarbonyl or phenyl, or
are phenyl which may carry one to three substituents selected from the group consisting of halogen and C₁-C₄-alkyl.

## Revendications

1. Utilisation de 2-(N-phénylamino)pyrimidines de formule I 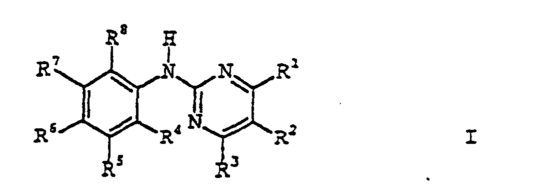 dans laquelle les symboles ont les significations suivantes :
R¹, R³, indépendamment l'un de l'autre, des groupes cyano, alkyle en C1-C8, alcényle en C2-C6, alcynyle en C2-C6, les groupes alkyle, alcényle et alcynyle pouvant eux-mêmes être substitués par des groupes cyano, des halogènes, des groupes alcoxy en C1-C4 ou (alcoxy en C1-C4)carbonyle, des groupes cycloalkyle en C3-C6 ou des groupes C(=NOR^{x})R^{y} ;
R^{x} : l'hydrogène, un groupe alkyle en C1-C8, alcényle en C2-C6, alcynyle en C2-C6, les groupes alkyle, alcényle et alcynyle pouvant eux-mêmes être substitués par des groupes cyano, des halogènes, des groupes alcoxy en C1-C4 ou phényle ;
R^{y} : l'hydrogène, un groupe alkyle en C1-C8, alcényle en C2-C6, alcynyle en C2-C6, les groupes alkyle, alcényle et alcynyle pouvant eux-mêmes être substitués par des groupes cyano, des halogènes ou des groupes alcoxy en C1-C4 ;
R² : un halogène, un groupe alkyle en C1-C8, alcényle en C2-C6, alcynyle en C2-C6, les groupes alkyle, alcényle et alcynyle pouvant eux-mêmes être substitués par des groupes cyano, des halogènes, des groupes alcoxy en C1-C4 ou (alcoxy en C1-C4)carbonyle ;
R⁴ à R⁸, indépendamment les uns des autres, l'hydrogène, des groupes cyano, des halogènes, des groupes R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} ou R^{a}O-N=C(R^{b}) ;
R^{a}, R^{b} : des groupes alkyle en C1-C4, alcényle en C3-C6 ou alcynyle en C3-C6 qui peuvent chacun être substitué par des groupes cyano, des halogènes, des groupes alcoxy en C1-C4, (alcoxy en Cl-C4)carbonyle ou phényle ;
ou bien
des groupes phényle qui peuvent porter un à trois substituants choisis parmi les halogènes et les groupes alkyle en C1-C4 ;
m : 0, 1 ou 2 ;
en tant que fongicides.

2. 2-(N-phénylamino)pyrimidines de formule I dans laquelle
R¹, R³ représentent chacun, indépendamment l'un de l'autre, un groupe cyano, alkyle en C1-C8, alcényle en C2-C6, alcynyle en C2-C6 ou cycloalkyle en C3-C8 ou un groupe C(=NOR^{x})R^{y} ;
R^{x} : l'hydrogène ou un groupe alkyle en C1-C8 ;
R^{y} : l'hydrogène ou un groupe alkyle en C1-C8 ;
R² : un halogène, un groupe alkyle en C1-C8, alcényle en C2-C6, alcynyle en C2-C6 ou halogénoalkyle en C1-C2 ;
R⁴, R⁶, R⁸, indépendamment les uns des autres, l'hydrogène, des groupes cyano, des halogènes, des groupes R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} ou R^{a}O-N=C(R^{b}) ;
R⁵, R⁷, indépendamment l'un de l'autre, l'hydrogène, des groupes cyano, R^{a}, R^{a}O, R^{a}S(O)ₘ, R^{a}O(C=O), R^{a}(C=O), R^{a}R^{b}N-(C=O), R^{a}HN-(C=O), H₂N-(C=O), R^{a}-(C=O)-NH, R^{a}-(C=O)-NR^{b} ou R^{a}O-N=C(R^{b}) ;
R^{a}, R^{b} : des groupes alkyle en C1-C4, alcényle en C3-C6 ou alcynyle en C3-C6 qui peuvent chacun être substitués par des groupes cyano, des halogènes, des groupes alcoxy en C1-C4, (alcoxy en Cl-C4)carbonyle ou phényle
ou bien
des groupes phényle qui peuvent porter un à trois des substituants choisis parmi les halogènes et les groupes alkyle en C1-C4, et
m est égal à 0, 1 ou 2.

3. 2-(N-phénylamino)pyrimidines de formule I selon la revendication 2 dans laquelle
R¹ représente un groupe méthyle ou cyclopropyle.

4. 2-(N-phénylamino)pyrimidines de formule I selon les revendications 2 et 3 dans laquelle
R⁴ à R⁸ représentent l'hydrogène.

5. 2-(N-phénylamino)pyrimidines de formule I selon les revendications 2 à 4 dans laquelle
R² représente un halogène ou un groupe méthyle.

6. 2-(N-phénylamino)pyrimidines de formule I selon les revendications 2 à 5, dans laquelle
R³ représente un groupe CH(=NOCH₃), CH(=NOC₂H₅), C(=NOCH₃)CH₃, C(=NOC₂H₅)CH₃, cyano ou méthyle.

7. 2-(N-phénylamino)pyrimidines de formule I dans laquelle
R¹ représente groupe méthyle ou cyclopropyle ;
R² représente un halogène ou un groupe méthyle ;
R³ représente le groupe trifluorométhyle ;
R⁴ et R⁸ représentent l'hydrogène ;
R⁵ et R⁷ représentent chacun, indépendamment l'un de l'autre, l'hydrogène, un groupe cyano, halogénoalcoxy en C1-C2, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} ou R^{a}O-N=C(R^{b}) ;
R⁶ indépendamment des autres, représente l'hydrogène, un groupe cyano, un halogène, un groupe halogénoalkyle en C1-C2, halogénoalcoxy en C1-C2, R^{a}, R^{a}O, R^{a}S(O)ₘ, R^{a}O-(C=O), R^{a}(C=O), R^{a}R^{b}N-(C=O), R^{a}HN-(C=O), H₂N-(C=O), R^{a}-(C=O)-NH, R^{a}-(C=O)-NR^{b} ou R^{a}O-N=C(R^{b}) ;
R^{a}, R^{b} représentent des groupes alkyle en C1-C4, alcényle en C3-C6 ou alcynyle en C3-C6, chacun d'eux pouvant être substitué par des groupes cyano, alcoxy en C1-C4, (alcoxy en C1-C4)carbonyle ou phényle, ou bien
des groupes phényle qui peuvent porter un à trois substituants choisis parmi les halogènes et les groupes alkyle en C1-C4.
